# EUROPEAN PATENT APPLICATION

(11) **EP 2 700 716 A1**
(43) Date of publication of application: **26.02.2014**
(21) Application number: 12774176.7
(22) Date of filing: 16.04.2012
(51) Int. Cl.: C12P 13/12, C12N 15/09

(54) **PROCESS FOR PRODUCING L-CYSTEINE**

(30) Priority: 18.04.2011 JP 2011092099
(71) Applicant: Ajinomoto Co., Inc., Chuo-ku, Tokyo 104-8315 (JP)
(72) Inventor: NONAKA, Gen, Kawasaki-shi Kanagawa 210-8681 (JP)
(74) Representative: Strehl Schübel-Hopf & Partner
(86) International application number: PCT/JP2012/060292
(87) International publication number: WO 2012/144472

(57) **Abstract**

A novel technique for improving L-cysteine-producing ability of bacteria is developed to provide a method for producing L-cysteine, a related substance thereof, or a mixture thereof. L-cysteine, a related substance thereof, or a mixture thereof is produced by culturing a bacterium belonging to the family *Enterobacteriaceae,* which has L-cysteine-producing ability and is modified so that expression of gene(s) selected from the group consisting of the *ygaZH* genes and the *ychE* gene is increased compared with a non-modified strain, in a medium, and collecting L-cysteine, a related substance thereof, or a mixture thereof from the medium.

SEQUENCE LISTING

<110>  Ajinomoto Co., Inc.

<120>  A method for producing L-cysteine

<130>  D318-12022

<150>  JP2011-092099
<151>  2011-04-18

<160>  17

<170>  PatentIn version 3.5

<210>  1
<211>  29
<212>  DNA
<213>  Artificial Sequence

<220>
<223>  ygaZH(Ec)SalI-F

<400>  1
acgcgtcgac atggaaagcc ctactccac                                          29

<210>  2
<211>  33
<212>  DNA
<213>  Artificial Sequence

<220>
<223>  ygaZH(Ec)XbaI-R

<400>  2
ctagtctaga ttatataatc gccatcactt tcc                                     33

<210>  3
<211>  36
<212>  DNA
<213>  Artificial Sequence

<220>
<223>  ychE(Ec)SalI-F

<400>  3
acgcgtcgac atgattcaga ccttttttga ttttcc                                   36

<210>  4
<211>  29
<212>  DNA
<213>  Artificial Sequence

<220>
<223>  ychE(Ec)XbaI-R

<400>  4
ctagtctaga ttaattaagc aggccgggg                                          29

<210>  5
<211>  32
<212>  DNA
<213>  Artificial Sequence

<220>
<223>  ychE(Pa)SalI-F

<400>  5
acgcgtcgac atgaatcctg cgttattaga tc                                    32


<210>  6
<211>  28
<212>  DNA
<213>  Artificial Sequence

<220>
<223>  ychE(Pa)XbaI-R

<400>  6
ctagtctaga ttacccaacc agaccggg                                         28


<210>  7
<211>  738
<212>  DNA
<213>  Escherichia coli

<220>
<221>  CDS
<222>  (1)..(738)

<400>  7
atg gaa agc cct act cca cag cct gct cct ggt tcg gcg acc ttc atg       48
Met Glu Ser Pro Thr Pro Gln Pro Ala Pro Gly Ser Ala Thr Phe Met
1               5                   10                  15
gaa gga tgc aaa gac agt tta ccg att gtt att agt tat att ccg gtg       96
Glu Gly Cys Lys Asp Ser Leu Pro Ile Val Ile Ser Tyr Ile Pro Val
            20                  25                  30
gcc ttt gcg ttc ggt ctg aat gcg acc cgt ctg gga ttc tct cct ctc      144
Ala Phe Ala Phe Gly Leu Asn Ala Thr Arg Leu Gly Phe Ser Pro Leu
        35                  40                  45
gaa agc gtt ttt ttc tcc tgc atc att tat gca ggc gcg agc cag ttc      192
Glu Ser Val Phe Phe Ser Cys Ile Ile Tyr Ala Gly Ala Ser Gln Phe
    50                  55                  60
gtc att acc gcg atg ctg gca gcc ggg agt agt ttg tgg att gct gca      240
Val Ile Thr Ala Met Leu Ala Ala Gly Ser Ser Leu Trp Ile Ala Ala
65                  70                  75                  80
ctg acc gtc atg gca atg gat gtt cgc cat gtg ttg tat ggc ccg tca      288
Leu Thr Val Met Ala Met Asp Val Arg His Val Leu Tyr Gly Pro Ser
                85                  90                  95
ctg cgt agc cgt att att cag cgt ctg caa aaa tcg aaa acc gcc ctg      336
Leu Arg Ser Arg Ile Ile Gln Arg Leu Gln Lys Ser Lys Thr Ala Leu
            100                 105                 110
tgg gcg ttt ggc ctg acg gat gag gtt ttt gcc gcc gca acc gca aaa      384
Trp Ala Phe Gly Leu Thr Asp Glu Val Phe Ala Ala Ala Thr Ala Lys
        115                 120                 125
ctg gta cgc aat aat cgc cgc tgg agc gag aac tgg atg atc ggc att      432
Leu Val Arg Asn Asn Arg Arg Trp Ser Glu Asn Trp Met Ile Gly Ile
    130                 135                 140
gcc ttc agt tca tgg tca tcg tgg gta ttt ggt acg gta ata ggg gca      480
Ala Phe Ser Ser Trp Ser Ser Trp Val Phe Gly Thr Val Ile Gly Ala

145                 150                 155                 160
ttc tcc ggc agc ggc ttg ctg caa ggt tat ccc gcc gtt gaa gct gca       528
Phe Ser Gly Ser Gly Leu Leu Gln Gly Tyr Pro Ala Val Glu Ala Ala
                165                 170                 175
tta ggt ttt atg ctt ccg gca ctc ttt atg agt ttc ctg ctc gcc tct       576
Leu Gly Phe Met Leu Pro Ala Leu Phe Met Ser Phe Leu Leu Ala Ser
            180                 185                 190
ttc cag cgc aaa caa tct ctt tgc gtt acc gca gcg tta gtt ggt gcc       624
Phe Gln Arg Lys Gln Ser Leu Cys Val Thr Ala Ala Leu Val Gly Ala
        195                 200                 205
ctt gca ggc gta acg cta ttt tct att ccc gtc gcc att ctg gca ggc       672
Leu Ala Gly Val Thr Leu Phe Ser Ile Pro Val Ala Ile Leu Ala Gly
    210                 215                 220
att gtc tgt ggc tgc ctc act gcg tta atc cag gca ttc tgg caa gga       720
Ile Val Cys Gly Cys Leu Thr Ala Leu Ile Gln Ala Phe Trp Gln Gly
225                 230                 235                 240
gcg ccc gat gag cta tga                                               738
Ala Pro Asp Glu Leu
                245


<210>  8
<211>  245
<212>  PRT
<213>  Escherichia coli

<400>  8
Met Glu Ser Pro Thr Pro Gln Pro Ala Pro Gly Ser Ala Thr Phe Met
1               5                   10                  15
Glu Gly Cys Lys Asp Ser Leu Pro Ile Val Ile Ser Tyr Ile Pro Val
            20                  25                  30
Ala Phe Ala Phe Gly Leu Asn Ala Thr Arg Leu Gly Phe Ser Pro Leu
        35                  40                  45
Glu Ser Val Phe Phe Ser Cys Ile Ile Tyr Ala Gly Ala Ser Gln Phe
    50                  55                  60
Val Ile Thr Ala Met Leu Ala Ala Gly Ser Ser Leu Trp Ile Ala Ala
65                  70                  75                  80
Leu Thr Val Met Ala Met Asp Val Arg His Val Leu Tyr Gly Pro Ser
                85                  90                  95
Leu Arg Ser Arg Ile Ile Gln Arg Leu Gln Lys Ser Lys Thr Ala Leu
            100                 105                 110
Trp Ala Phe Gly Leu Thr Asp Glu Val Phe Ala Ala Ala Thr Ala Lys
        115                 120                 125
Leu Val Arg Asn Asn Arg Arg Trp Ser Glu Asn Trp Met Ile Gly Ile
    130                 135                 140
Ala Phe Ser Ser Trp Ser Ser Trp Val Phe Gly Thr Val Ile Gly Ala
145                 150                 155                 160
Phe Ser Gly Ser Gly Leu Leu Gln Gly Tyr Pro Ala Val Glu Ala Ala
                165                 170                 175
Leu Gly Phe Met Leu Pro Ala Leu Phe Met Ser Phe Leu Leu Ala Ser
            180                 185                 190
Phe Gln Arg Lys Gln Ser Leu Cys Val Thr Ala Ala Leu Val Gly Ala
        195                 200                 205
Leu Ala Gly Val Thr Leu Phe Ser Ile Pro Val Ala Ile Leu Ala Gly
    210                 215                 220
Ile Val Cys Gly Cys Leu Thr Ala Leu Ile Gln Ala Phe Trp Gln Gly
225                 230                 235                 240
Ala Pro Asp Glu Leu
                245


<210>  9
<211>  336

<212>  DNA
<213>  Escherichia coli

<220>
<221>  CDS
<222>  (1)..(336)

<400>  9
atg agc tat gag gtt ctg ctg ctt ggg tta cta gtt ggc gtg gcg aat      48
Met Ser Tyr Glu Val Leu Leu Leu Gly Leu Leu Val Gly Val Ala Asn
1               5                   10                  15
tat tgc ttc cgc tat ttg ccg ctg cgc ctg cgt gtg ggt aat gcc cgc      96
Tyr Cys Phe Arg Tyr Leu Pro Leu Arg Leu Arg Val Gly Asn Ala Arg
            20                  25                  30
cca acc aaa cgt ggc gcg gta ggt att ttg ctc gac acc att ggc atc      144
Pro Thr Lys Arg Gly Ala Val Gly Ile Leu Leu Asp Thr Ile Gly Ile
        35                  40                  45
gcc tcg ata tgc gct ctg ctg gtt gtc tct acc gca cca gaa gtg atg      192
Ala Ser Ile Cys Ala Leu Leu Val Val Ser Thr Ala Pro Glu Val Met
    50                  55                  60
cac gat aca cgc cgt ttc gtg ccc acg ctg gtc ggc ttc gcg gta ctg      240
His Asp Thr Arg Arg Phe Val Pro Thr Leu Val Gly Phe Ala Val Leu
65                  70                  75                  80
ggt gcc agt ttc tat aaa aca cgc agc att atc atc cca aca ctg ctt      288
Gly Ala Ser Phe Tyr Lys Thr Arg Ser Ile Ile Ile Pro Thr Leu Leu
                85                  90                  95
agt gcg ctg gcc tat ggg ctc gcc tgg aaa gtg atg gcg att ata taa      336
Ser Ala Leu Ala Tyr Gly Leu Ala Trp Lys Val Met Ala Ile Ile
            100                 105                 110


<210>  10
<211>  111
<212>  PRT
<213>  Escherichia coli

<400>  10
Met Ser Tyr Glu Val Leu Leu Leu Gly Leu Leu Val Gly Val Ala Asn
1               5                   10                  15
Tyr Cys Phe Arg Tyr Leu Pro Leu Arg Leu Arg Val Gly Asn Ala Arg
            20                  25                  30
Pro Thr Lys Arg Gly Ala Val Gly Ile Leu Leu Asp Thr Ile Gly Ile
        35                  40                  45
Ala Ser Ile Cys Ala Leu Leu Val Val Ser Thr Ala Pro Glu Val Met
    50                  55                  60
His Asp Thr Arg Arg Phe Val Pro Thr Leu Val Gly Phe Ala Val Leu
65                  70                  75                  80
Gly Ala Ser Phe Tyr Lys Thr Arg Ser Ile Ile Ile Pro Thr Leu Leu
                85                  90                  95
Ser Ala Leu Ala Tyr Gly Leu Ala Trp Lys Val Met Ala Ile Ile
            100                 105                 110


<210>  11
<211>  1063
<212>  DNA
<213>  Escherichia coli

<400>  11
atggaaagcc ctactccaca gcctgctcct ggttcggcga ccttcatgga aggatgcaaa      60
gacagtttac cgattgttat tagttatatt ccggtggcct ttgcgttcgg tctgaatgcg      120
acccgtctgg gattctctcc tctcgaaagc gtttttttct cctgcatcat ttatgcaggc      180
gcgagccagt tcgtcattac cgcgatgctg gcagccggga gtagtttgtg gattgctgca      240

ctgaccgtca tggcaatgga tgttcgccat gtgttgtatg gcccgtcact gcgtagccgt 300
attattcagc gtctgcaaaa atcgaaaacc gccctgtggg cgtttggcct gacggatgag 360
gtttttgccg ccgcaaccgc aaaactggta cgcaataatc gccgctggag cgagaactgg 420
atgatcggca ttgccttcag ttcatggtca tcgtgggtat ttggtacggt aataggggca 480
ttctccggca gcggcttgct gcaaggttat cccgccgttg aagctgcatt aggttttatg 540
cttccggcac tctttatgag tttcctgctc gcctctttcc agcgcaaaca atctctttgc 600
gttaccgcag cgttagttgg tgcccttgca ggcgtaacgc tattttctat tcccgtcgcc 660
attctggcag gcattgtctg tggctgcctc actgcgttaa tccaggcatt ctggcaagga 720
gcgcccgatg agctatgagg ttctgctgct tgggttacta gttggcgtgg cgaattattg 780
cttccgctat ttgccgctgc gcctgcgtgt gggtaatgcc cgcccaacca aacgtggcgc 840
ggtaggtatt ttgctcgaca ccattggcat cgcctcgata tgcgctctgc tggttgtctc 900
taccgcacca gaagtgatgc acgatacacg ccgtttcgtg cccacgctgg tcggcttcgc 960
ggtactgggt gccagtttct ataaaacacg cagcattatc atcccaacac tgcttagtgc 1020
gctggcctat gggctcgcct ggaaagtgat ggcgattata taa 1063

<210> 12
<211> 648
<212> DNA
<213> Escherichia coli

<220>
<221> CDS
<222> (1)..(648)

<400> 12
gtg att cag acc ttt ttt gat ttt ccc gtt tac ttc aaa ttt ttc atc 48
Met Ile Gln Thr Phe Phe Asp Phe Pro Val Tyr Phe Lys Phe Phe Ile
1               5                   10                  15
ggg tta ttt gcg ctg gtc aac ccg gta ggg att att ccc gtc ttt atc 96
Gly Leu Phe Ala Leu Val Asn Pro Val Gly Ile Ile Pro Val Phe Ile
            20                  25                  30
agc atg acc agt tat cag aca gcg gca gcg cga aac aaa act aac ctt 144
Ser Met Thr Ser Tyr Gln Thr Ala Ala Ala Arg Asn Lys Thr Asn Leu
        35                  40                  45
aca gcc aac ctg tct gtg gcc att atc ttg tgg atc tcg ctt ttt ctc 192
Thr Ala Asn Leu Ser Val Ala Ile Ile Leu Trp Ile Ser Leu Phe Leu
    50                  55                  60
ggc gac acg att cta caa ctt ttt ggt ata tca att gat tcg ttc cgt 240
Gly Asp Thr Ile Leu Gln Leu Phe Gly Ile Ser Ile Asp Ser Phe Arg
65                  70                  75                  80
atc gcc ggg ggt atc ctg gtg gtg aca ata gcg atg tcg atg atc agc 288
Ile Ala Gly Gly Ile Leu Val Val Thr Ile Ala Met Ser Met Ile Ser
                85                  90                  95
ggc aag ctt ggc gag gat aaa cag aac aag caa gaa aaa tca gaa acc 336
Gly Lys Leu Gly Glu Asp Lys Gln Asn Lys Gln Glu Lys Ser Glu Thr
            100                 105                 110
gcg gta cgt gaa agc att ggt gtg gtg cca ctg gcg ttg ccg ttg atg 384
Ala Val Arg Glu Ser Ile Gly Val Val Pro Leu Ala Leu Pro Leu Met
        115                 120                 125
gcg ggg cca ggg gcg atc agt tct acc atc gtc tgg ggt acg cgt tat 432
Ala Gly Pro Gly Ala Ile Ser Ser Thr Ile Val Trp Gly Thr Arg Tyr
    130                 135                 140
cac agc att agc tat ctg ttt ggt ttc ttt gtg gct att gca ttg ttc 480
His Ser Ile Ser Tyr Leu Phe Gly Phe Phe Val Ala Ile Ala Leu Phe
145                 150                 155                 160
gct tta tgt tgt tgg gga ttg ttc cgc atg gca ccg tgg ctg gta cgg 528
Ala Leu Cys Cys Trp Gly Leu Phe Arg Met Ala Pro Trp Leu Val Arg
                165                 170                 175
gtt tta cgc cag acc ggc atc aac gtg att acg cgt att atg ggg cta 576
Val Leu Arg Gln Thr Gly Ile Asn Val Ile Thr Arg Ile Met Gly Leu
            180                 185                 190
ttg ctg atg gca ttg ggg att gaa ttt atc gtt act ggt att aag ggg 624

Leu Leu Met Ala Leu Gly Ile Glu Phe Ile Val Thr Gly Ile Lys Gly
        195                 200                 205
att ttc ccc ggc ctg ctt aat taa                                     648
Ile Phe Pro Gly Leu Leu Asn
    210                 215

<210>  13
<211>  215
<212>  PRT
<213>  Escherichia coli

<400>  13
Met Ile Gln Thr Phe Phe Asp Phe Pro Val Tyr Phe Lys Phe Phe Ile
1               5                   10                  15
Gly Leu Phe Ala Leu Val Asn Pro Val Gly Ile Ile Pro Val Phe Ile
            20                  25                  30
Ser Met Thr Ser Tyr Gln Thr Ala Ala Ala Arg Asn Lys Thr Asn Leu
        35                  40                  45
Thr Ala Asn Leu Ser Val Ala Ile Ile Leu Trp Ile Ser Leu Phe Leu
    50                  55                  60
Gly Asp Thr Ile Leu Gln Leu Phe Gly Ile Ser Ile Asp Ser Phe Arg
65                  70                  75                  80
Ile Ala Gly Gly Ile Leu Val Val Thr Ile Ala Met Ser Met Ile Ser
                85                  90                  95
Gly Lys Leu Gly Glu Asp Lys Gln Asn Lys Gln Glu Lys Ser Glu Thr
            100                 105                 110
Ala Val Arg Glu Ser Ile Gly Val Val Pro Leu Ala Leu Pro Leu Met
        115                 120                 125
Ala Gly Pro Gly Ala Ile Ser Ser Thr Ile Val Trp Gly Thr Arg Tyr
    130                 135                 140
His Ser Ile Ser Tyr Leu Phe Gly Phe Phe Val Ala Ile Ala Leu Phe
145                 150                 155                 160
Ala Leu Cys Cys Trp Gly Leu Phe Arg Met Ala Pro Trp Leu Val Arg
                165                 170                 175
Val Leu Arg Gln Thr Gly Ile Asn Val Ile Thr Arg Ile Met Gly Leu
            180                 185                 190
Leu Leu Met Ala Leu Gly Ile Glu Phe Ile Val Thr Gly Ile Lys Gly
        195                 200                 205
Ile Phe Pro Gly Leu Leu Asn
    210                 215

<210>  14
<211>  648
<212>  DNA
<213>  Pantoea ananatis

<220>
<221>  CDS
<222>  (1)..(648)

<400>  14
gtg aat cct gcg tta tta gat ctg tct ggc tat atc aag ttt ttc gta      48
Met Asn Pro Ala Leu Leu Asp Leu Ser Gly Tyr Ile Lys Phe Phe Val
1               5                   10                  15
ggc ctg ttt gca ctg gtt aat ccc atc ggc atc att ccg gta ttt att      96
Gly Leu Phe Ala Leu Val Asn Pro Ile Gly Ile Ile Pro Val Phe Ile
            20                  25                  30
agc atg acc cgc tat cag ggc gtg gca gaa cgc aat aaa acc aac ctg      144
Ser Met Thr Arg Tyr Gln Gly Val Ala Glu Arg Asn Lys Thr Asn Leu
        35                  40                  45
acg gcc aac ctc gcg gtg gcg att att ctc tgg acc tcg ctg ttt ctg      192

Thr Ala Asn Leu Ala Val Ala Ile Ile Leu Trp Thr Ser Leu Phe Leu
    50                  55                  60
ggc gat gca atc ctg cat gtc ttc ggt atc tct atc gac tcg ttt cgc      240
Gly Asp Ala Ile Leu His Val Phe Gly Ile Ser Ile Asp Ser Phe Arg
65                  70                  75                  80
att gcg ggc ggg att ctg gtg gtg acc atc gct atg tcg atg att agc      288
Ile Ala Gly Gly Ile Leu Val Val Thr Ile Ala Met Ser Met Ile Ser
                85                  90                  95
ggc aag ctg ggc gag gat aag caa aat aag cag gaa aaa acg gaa acg      336
Gly Lys Leu Gly Glu Asp Lys Gln Asn Lys Gln Glu Lys Thr Glu Thr
            100                 105                 110
gca att cgg gaa agt att ggc gtg gtt ccc ctt gcg ctg ccg ctc atg      384
Ala Ile Arg Glu Ser Ile Gly Val Val Pro Leu Ala Leu Pro Leu Met
        115                 120                 125
gcg ggc cca ggc gcc atc agt tcg act atc gtc tgg agc aca cgt tat      432
Ala Gly Pro Gly Ala Ile Ser Ser Thr Ile Val Trp Ser Thr Arg Tyr
    130                 135                 140
aac aac tgg ctc aac ctt ttg ggt ttt acc gtt gcg att gct gtt ttc      480
Asn Asn Trp Leu Asn Leu Leu Gly Phe Thr Val Ala Ile Ala Val Phe
145                 150                 155                 160
gcc ttt agc tgt tgg ctg ctg ttc cgc gct gcg cct tta atg gtc agg      528
Ala Phe Ser Cys Trp Leu Leu Phe Arg Ala Ala Pro Leu Met Val Arg
                165                 170                 175
ctg tta ggg cag aca ggg att aac gtg att acc cgt atc atg gga ttg      576
Leu Leu Gly Gln Thr Gly Ile Asn Val Ile Thr Arg Ile Met Gly Leu
            180                 185                 190
ctg tta atg gcg cta ggt att gaa ttt gtg gtg act ggc atc aag gca      624
Leu Leu Met Ala Leu Gly Ile Glu Phe Val Val Thr Gly Ile Lys Ala
        195                 200                 205
att ttt ccc ggt ctg gtt ggg taa                                      648
Ile Phe Pro Gly Leu Val Gly
    210                 215


<210>  15
<211>  215
<212>  PRT
<213>  Pantoea ananatis

<400>  15
Met Asn Pro Ala Leu Leu Asp Leu Ser Gly Tyr Ile Lys Phe Phe Val
1               5                   10                  15
Gly Leu Phe Ala Leu Val Asn Pro Ile Gly Ile Ile Pro Val Phe Ile
            20                  25                  30
Ser Met Thr Arg Tyr Gln Gly Val Ala Glu Arg Asn Lys Thr Asn Leu
        35                  40                  45
Thr Ala Asn Leu Ala Val Ala Ile Ile Leu Trp Thr Ser Leu Phe Leu
    50                  55                  60
Gly Asp Ala Ile Leu His Val Phe Gly Ile Ser Ile Asp Ser Phe Arg
65                  70                  75                  80
Ile Ala Gly Gly Ile Leu Val Val Thr Ile Ala Met Ser Met Ile Ser
                85                  90                  95
Gly Lys Leu Gly Glu Asp Lys Gln Asn Lys Gln Glu Lys Thr Glu Thr
            100                 105                 110
Ala Ile Arg Glu Ser Ile Gly Val Val Pro Leu Ala Leu Pro Leu Met
        115                 120                 125
Ala Gly Pro Gly Ala Ile Ser Ser Thr Ile Val Trp Ser Thr Arg Tyr
    130                 135                 140
Asn Asn Trp Leu Asn Leu Leu Gly Phe Thr Val Ala Ile Ala Val Phe
145                 150                 155                 160
Ala Phe Ser Cys Trp Leu Leu Phe Arg Ala Ala Pro Leu Met Val Arg
                165                 170                 175
Leu Leu Gly Gln Thr Gly Ile Asn Val Ile Thr Arg Ile Met Gly Leu

180                 185                 190
Leu Leu Met Ala Leu Gly Ile Glu Phe Val Val Thr Gly Ile Lys Ala
        195                 200                 205
Ile Phe Pro Gly Leu Val Gly
    210                 215

<210>  16
<211>  1422
<212>  DNA
<213>  Escherichia coli

<220>
<221>  CDS
<222>  (301)..(1122)

<400>  16
tcggtcaggg catggatgta caaagcgcgc aggagaagat tggtcaggtg gtggaaggct      60
accgcaatac gaaagaagtc cgcgaactgg cgcatcgctt cggcgttgaa atgccaataa     120
ccgaggaaat ttatcaagta ttatattgcg gaaaaaacgc gcgcgaggca gcattgactt     180
tactaggtcg tgcacgcaag gacgagcgca gcagccacta accccaggga acctttgtta     240
ccgctatgac ccggcccgcg cagaacgggc cggtcattat ctcatcgtgt ggagtaagca     300
atg tcg tgt gaa gaa ctg gaa att gtc tgg aac aat att aaa gcc gaa       348
Met Ser Cys Glu Glu Leu Glu Ile Val Trp Asn Asn Ile Lys Ala Glu
1               5                   10                  15
gcc aga acg ctg gcg gac tgt gag cca atg ctg gcc agt ttt tac cac       396
Ala Arg Thr Leu Ala Asp Cys Glu Pro Met Leu Ala Ser Phe Tyr His
            20                  25                  30
gcg acg cta ctc aag cac gaa aac ctt ggc agt gca ctg agc tac atg       444
Ala Thr Leu Leu Lys His Glu Asn Leu Gly Ser Ala Leu Ser Tyr Met
        35                  40                  45
ctg gcg aac aag ctg tca tcg cca att atg cct gct att gct atc cgt       492
Leu Ala Asn Lys Leu Ser Ser Pro Ile Met Pro Ala Ile Ala Ile Arg
    50                  55                  60
gaa gtg gtg gaa gaa gcc tac gcc gct gac ccg gaa atg atc gcc tct       540
Glu Val Val Glu Glu Ala Tyr Ala Ala Asp Pro Glu Met Ile Ala Ser
65                  70                  75                  80
gcg gcc tgt gat att cag gcg gtg cgt acc cgc gac ccg gca gtc gat       588
Ala Ala Cys Asp Ile Gln Ala Val Arg Thr Arg Asp Pro Ala Val Asp
                85                  90                  95
aaa tac tca acc ccg ttg tta tac ctg aag ggt ttt cat gcc ttg cag       636
Lys Tyr Ser Thr Pro Leu Leu Tyr Leu Lys Gly Phe His Ala Leu Gln
            100                 105                 110
gcc tat cgc atc ggt cac tgg ttg tgg aat cag ggg cgt cgc gca ctg       684
Ala Tyr Arg Ile Gly His Trp Leu Trp Asn Gln Gly Arg Arg Ala Leu
        115                 120                 125
gca atc ttt ctg caa aac cag gtt tct gtg acg ttc cag gtc gat att       732
Ala Ile Phe Leu Gln Asn Gln Val Ser Val Thr Phe Gln Val Asp Ile
    130                 135                 140
cac ccg gca gca aaa att ggt cgc ggt atc atg ctt gac cac gcg aca       780
His Pro Ala Ala Lys Ile Gly Arg Gly Ile Met Leu Asp His Ala Thr
145                 150                 155                 160
ggc atc gtc gtt ggt gaa acg gcg gtg att gaa aac gac gta tcg att       828
Gly Ile Val Val Gly Glu Thr Ala Val Ile Glu Asn Asp Val Ser Ile
                165                 170                 175
ctg caa tct gtg acg ctt ggc ggt acg ggt aaa tct ggt ggt gac cgt       876
Leu Gln Ser Val Thr Leu Gly Gly Thr Gly Lys Ser Gly Gly Asp Arg
            180                 185                 190
cac ccg aaa att cgt gaa ggt gtg atg att ggc gcg ggc gcg aaa atc       924
His Pro Lys Ile Arg Glu Gly Val Met Ile Gly Ala Gly Ala Lys Ile
        195                 200                 205
ctc ggc aat att gaa gtt ggg cgc ggc gcg aag att ggc gca ggt tcc       972
Leu Gly Asn Ile Glu Val Gly Arg Gly Ala Lys Ile Gly Ala Gly Ser

210                 215                 220
gtg gtg ctg caa ccg gtg ccg ccg cat acc acc gcc gct ggc gtt ccg      1020
Val Val Leu Gln Pro Val Pro Pro His Thr Thr Ala Ala Gly Val Pro
225                 230                 235                 240
gct cgt att gtc ggt aaa cca gac agc gat aag cca tca atg gat atg      1068
Ala Arg Ile Val Gly Lys Pro Asp Ser Asp Lys Pro Ser Met Asp Met
                245                 250                 255
gac cag cat ttc aac ggt att aac cat aca ttt gag tat ggg gat ggg      1116
Asp Gln His Phe Asn Gly Ile Asn His Thr Phe Glu Tyr Gly Asp Gly
            260                 265                 270
atc taa tgtcctgtga tcgtgccgga tgcgatgtaa tcatctatcc ggcctacagt      1172
Ile

aactaatctc tcaataccgc tcccggatac cccaactgcc gccaggcttc atacaccact      1232
accgacaccg cattggacag attcatgctg cggctgtccg gcaccatcgg aatgcgaatt      1292
ttttgttcag cgggcagggc atcaagaatg ctcgctggca ggccgcgtgt ttccgggccg      1352
aacatcagat aatcgccatc ctgatagctt acggcgctgt gagcaggtgt acctttcgtg      1412
gtgagggcga                                                            1422


<210>  17
<211>  273
<212>  PRT
<213>  Escherichia coli

<400>  17
Met Ser Cys Glu Glu Leu Glu Ile Val Trp Asn Asn Ile Lys Ala Glu
1               5                   10                  15
Ala Arg Thr Leu Ala Asp Cys Glu Pro Met Leu Ala Ser Phe Tyr His
            20                  25                  30
Ala Thr Leu Leu Lys His Glu Asn Leu Gly Ser Ala Leu Ser Tyr Met
        35                  40                  45
Leu Ala Asn Lys Leu Ser Ser Pro Ile Met Pro Ala Ile Ala Ile Arg
    50                  55                  60
Glu Val Val Glu Glu Ala Tyr Ala Ala Asp Pro Glu Met Ile Ala Ser
65                  70                  75                  80
Ala Ala Cys Asp Ile Gln Ala Val Arg Thr Arg Asp Pro Ala Val Asp
                85                  90                  95
Lys Tyr Ser Thr Pro Leu Leu Tyr Leu Lys Gly Phe His Ala Leu Gln
            100                 105                 110
Ala Tyr Arg Ile Gly His Trp Leu Trp Asn Gln Gly Arg Arg Ala Leu
        115                 120                 125
Ala Ile Phe Leu Gln Asn Gln Val Ser Val Thr Phe Gln Val Asp Ile
    130                 135                 140
His Pro Ala Ala Lys Ile Gly Arg Gly Ile Met Leu Asp His Ala Thr
145                 150                 155                 160
Gly Ile Val Val Gly Glu Thr Ala Val Ile Glu Asn Asp Val Ser Ile
                165                 170                 175
Leu Gln Ser Val Thr Leu Gly Gly Thr Gly Lys Ser Gly Gly Asp Arg
            180                 185                 190
His Pro Lys Ile Arg Glu Gly Val Met Ile Gly Ala Gly Ala Lys Ile
        195                 200                 205
Leu Gly Asn Ile Glu Val Gly Arg Gly Ala Lys Ile Gly Ala Gly Ser
    210                 215                 220
Val Val Leu Gln Pro Val Pro Pro His Thr Thr Ala Ala Gly Val Pro
225                 230                 235                 240
Ala Arg Ile Val Gly Lys Pro Asp Ser Asp Lys Pro Ser Met Asp Met
                245                 250                 255
Asp Gln His Phe Asn Gly Ile Asn His Thr Phe Glu Tyr Gly Asp Gly
            260                 265                 270
Ile

## Description

### Technical Field

The present invention relates to a bacterium suitable for the production of L-cysteine or a related substance thereof, and a method for producing L-cysteine or a related substance thereof utilizing such a bacterium. L-cysteine and related substances thereof are used in various fields such as those of seasonings, food additives, feed additives, chemicals, and drugs.

### Background Art

L-cysteine is conventionally obtained by extraction from keratin-containing substances such as hairs, horns, and feathers or conversion of DL-2-aminothiazoline-4-carboxylic acid as a precursor using a bacterial enzyme. It is also planned to produce L-cysteine in a large scale by an immobilized enzyme method utilizing a novel enzyme. Furthermore, it is also attempted to produce L-cysteine by fermentation utilizing a microorganism.

As microorganisms preferably used for the production of L-cysteine, there are known microorganisms of which L-cysteine-producing ability is improved by enhancing activity of an enzyme of the L-cysteine biosynthesis pathway or an enzyme involved in synthesis of a compound serving as a substrate of that pathway, such as L-serine. For example, as a microorganism having L-cysteine-producing ability, there is known a coryneform bacterium of which intracellular serine acetyltransferase (EC 2.3.1.30, henceforth also referred to as "SAT") activity is increased (Patent document 1). There is also known a technique of enhancing L-cysteine-producing ability by incorporating a mutant serine acetyltransferase of which feedback inhibition by L-cysteine is attenuated (Patent documents 2 to 4). Furthermore, there is also known a technique of enhancing L-cysteine-producing ability by incorporating a mutant 3-phosphoglycerate dehydrogenase of which feedback inhibition by serine is attenuated (Patent documents 5 and 6).

Furthermore, L-cysteine-producing ability of a microorganism can also be improved by suppressing the L-cysteine decomposition system. As bacteria of which L-cysteine-producing ability is enhanced by suppressing the L-cysteine decomposition system, there are known coryneform bacteria or *Escherichia* bacteria in which activity of cystathionine-β-lyase (Patent document 2), tryptophanase (Patent document 7), or O-acetylserine sulfhydrylase B (Patent document 8) is decreased or deleted.

Moreover, L-cysteine-producing ability of a microorganism can also be improved by enhancing L-cysteine-secreting ability. For example, it is known that the *ydeD* gene coding for the YdeD protein (Non-patent document 1, Patent document 9) and the *yfiK* gene coding for the YfiK protein (Non-patent document 2, Patent document 10) participate in secretion of metabolic products of the L-cysteine pathway. Furthermore, there are also known techniques of enhancing L-cysteine-producing ability by increasing expression of the *mar* locus, acr locus, cmr locus, *mex* locus, *bmr* locus, or *qacA* locus (Patent document 9), or *emrAB, emrKY, yojIH, acrEF, bcr,* or *cusA* gene (Patent document 11), which is a gene coding for a protein suitable for secreting a cytotoxic substance. There is also known a technique of enhancing L-cysteine-producing ability by increasing expression of the *yeaS* gene coding for the YeaS protein or a mutant gene thereof (Patent document 12).

O-acetylserine is a precursor of L-cysteine, and therefore, O-acetylserine and L-cysteine share a common biosynthetic pathway. Accordingly, enhancement of the L-cysteine biosynthetic pathway is also effective for production of O-acetylserine. For example, there are known methods for producing O-acetylserine using a mutant serine acetyltransferase (Patent document 4) or a mutant 3-phosphoglycerate dehydrogenase (Patent documents 5 and 6).

Furthermore, O-acetylserine-producing ability of a microorganism can also be improved by enhancing O-acetylserine-secreting ability. For example, it is known that O-acetylserine-producing ability can be improved by enhancing the *ydeD* gene coding for the YdeD protein (Non-patent document 1, Patent document 9) or the *yfiK* gene coding for the YfiK protein (Non-patent document 2, Patent document 10).

O-acetylserine is easily converted into N-acetylserine by a spontaneous reaction in a neutral to alkaline pH region. For preventing such conversion into N-acetylserine, there is known a method of producing O-acetylserine in a pH region in which O-acetylserine stably exists (Patent document 13).

It is known that the YgaZ protein encoded by the *ygaZ* gene and the YgaH protein encoded by the *ygaH* gene form a complex (YgaZH), and they are L-valine secretion membrane proteins belonging to the branched chain amino acid exporter (LIV-E) family. It is considered that the *ygaZ* gene and the *ygaH* gene form an operon, and gene expression of the *ygaZH* operon is regulated by the transcription factor Lrp (Non-patent document 3). Furthermore, it is also known that expression of the *ygaZH* operon is induced under an alkaline environment (Non-patent document 4). It has also been reported that, by increasing gene expression amounts of the *ygaZH* genes in *Escherichia coli,* resistance to high concentration L-valine, L-norleucine, L-o-fluorophenylalanine, DL-o-fluorophenylalanine, 3,4-dihydroproline, DL-thiaisoleucine, DL-o-methylserine, or 4-azaleucine is improved compared with a control strain, and by increasing gene expression amounts of the genes in an L-amino acid-producing bacterium, productivity for L-valine, L-threonine, L-proline, or L-methionine is improved (Patent document 14).

The YchE protein encoded by the *ychE* gene is registered at the database EcoCyc (http://biocyc.org/ECOLI) as a membrane protein of which function is unknown. Furthermore, it is predicted that the YchE protein is a six-transmembrane protein, and the C-terminus thereof faces the periplasm (Non-patent document 5). As *ychE* paralogues, *yhgN* and *marC* are known, and it is thought that *marC* is involved in multiple-drug resistance. However, difference in sensitivity to various drugs is not observed between a triple-deficient strain of these genes and a wild strain, and therefore the relation between these genes and drug resistance is still unknown (Non-patent document 6). It has also been reported that, by increasing gene expression amount of the *ychE* gene in *Escherichia coli,* resistance to high concentration DL-o-methylserine and homoserine is improved compared with a control strain, and by increasing gene expression amount of that gene in an L-amino acid-producing bacterium, productivity for L-threonine or L-valine is improved (Patent document 14).

As described above, although it is known that production of several kinds of amino acids can be improved by increasing expression amount of the *ygaZH* genes or the *ychE* gene, relation between expression of the *ygaZH* genes or the *ychE* gene and L-cysteine production is not known.

### Prior Art References

### Patent documents

Patent document 1: Japanese Patent Laid-open (Kokai) No. 2002-233384
Patent document 2: Japanese Patent Laid-open (Kokai) No. 11-155571
Patent document 3: U.S. Patent Published Application No. 20050112731
Patent document 4: U.S. Patent No. 6,218,168
Patent document 5: U.S. Patent No. 5,856,148
Patent document 6: U.S. Patent Published Application No. 20050009162
Patent document 7: Japanese Patent Laid-open (Kokai) No. 2003-169668
Patent document 8: Japanese Patent Laid-open (Kokai) No. 2005-245311
Patent document 9: U.S. Patent No. 5,972,663
Patent document 10: Japanese Patent Laid-open (Kokai) No. 2004-49237
Patent document 11: Japanese Patent Laid-open (Kokai) No. 2005-287333
Patent document 12: Japanese Patent Laid-open (Kokai) No. 2010-187552
Patent document 13: U.S. Patent Published Application No. 20020146783
Patent document 14: U.S. Patent No. 7,476,531

### Non-patent documents

Non-patent document 1: Dassler et al., Mol. Microbiol., 36, 1101-1112 (2000)
Non-patent document 2: Franke I. et al., J Bacteriol., 2003 Feb, 185(4):1161-6
Non-patent Document 3: Park JH. et al., Proc. Natl. Acad. Sci. USA, 2007 May 8, 104(19):7797-802
Non-patent document 4: Hayes ET. et al., BMC Microbiol., 2006 Oct, 6;6:89
Non-patent document 5: Daley DO. et al., Science, 2005 May 27, 308(5726):1321-3
Non-patent document 6: McDermott PF. et al., Antimicrob. Agents Chemother., 2008 Jan, 52(1):382-3. Epub 2007 Oct 22

### Disclosure of the Invention

### Object to be Achieved by the Invention

An object of the present invention is to develop a novel technique for improving L-cysteine-producing ability of bacteria, and thereby provide an L-cysteine-producing bacterium and a method for producing L-cysteine, a related substance thereof, or a mixture thereof using the bacterium.

### Means for Achieving the Object

The inventors of the present invention conducted various researches in order to achieve the aforementioned object. As a result, they found that L-cysteine-producing ability of a bacterium could be improved by increasing expression amount of the *ygaZH* genes or the ychE gene, and accomplished the present invention. The present invention thus relates to the followings.

[1] A method for producing L-cysteine, a related substance thereof, or a mixture thereof, comprising:
   culturing a bacterium belonging to the family *Enterobacteriaceae,* which has L-cysteine-producing ability and is modified so that expression of gene(s) selected from the group consisting of *ygaZH* genes and ychE gene is increased compared with a non-modified strain, in a medium, and
   collecting L-cysteine, a related substance thereof, or a mixture thereof from the medium.
[2] The method as mentioned above, wherein the bacterium is modified so that the L-cysteine biosynthesis system is enhanced.
[3] The method as mentioned above, wherein the bacterium has a mutant serine acetyltransferase of which feedback inhibition by L-cysteine is attenuated or eliminated.
[4] The method as mentioned above, wherein the *ygaZH* genes code for a protein selected from the group consisting of (A) and (B) mentioned below, and a protein selected from the group consisting of (C) and (D) mentioned below, respectively:
   (A) a protein having the amino acid sequence of SEQ ID NO: 8;
   (B) a protein having the amino acid sequence of SEQ ID NO: 8 but which includes 1 to 10 amino acid substitutions, deletions, insertions, or additions, and having an activity of improving L-cysteine-producing ability of the *Enterobacteriaceae* bacterium compared with a non-modified strain when expression thereof is increased in the bacterium;
   (C) a protein having the amino acid sequence of SEQ ID NO: 10;
   (D) a protein having the amino acid sequence of SEQ ID NO: 10 but which includes 1 to 10 amino acid substitutions, deletions, insertions, or additions, and having an activity of improving L-cysteine-producing ability of the *Enterobacteriaceae* bacterium compared with a non-modified strain when expression thereof is increased in the bacterium.
[5] The method as mentioned above, wherein the *ychE* gene codes for a protein selected from the group consisting of (E) and (F) mentioned below:
   (E) a protein having the amino acid sequence of SEQ ID NO: 13 or 15;
   (E) a protein having the amino acid sequence of SEQ ID NO: 13 or 15 but which includes 1 to 10 amino acid substitutions, deletions, insertions, or additions, and having an activity of improving L-cysteine-producing ability of the *Enterobacteriaceae* bacterium compared with a non-modified strain when expression thereof is increased in the bacterium.
[6] The method as mentioned above, wherein the *ygaZH* genes are a DNA selected from the group consisting of (a) and (b) mentioned below, and a DNA selected from the group consisting of (c) and (d) mentioned below, respectively:
   (a) a gene having the nucleotide sequence of SEQ ID NO: 7;
   (b) a DNA that hybridizes with a sequence complementary to the nucleotide sequence of SEQ ID NO: 7 or a probe that can be prepared from the nucleotide sequence under stringent conditions, and codes for a protein having an activity of improving L-cysteine-producing ability of the *Enterobacteriaceae* bacterium compared with a non-modified strain when expression thereof is increased in the bacterium;
   (c) a gene having the nucleotide sequence of SEQ ID NO: 9;
   (d) a DNA that hybridizes with a sequence complementary to the nucleotide sequence of SEQ ID NO: 9 or a probe that can be prepared from the nucleotide sequence under stringent conditions, and codes for a protein having an activity of improving L-cysteine-producing ability of the *Enterobacteriaceae* bacterium compared with a non-modified strain when expression thereof is increased in the bacterium.
[7] The method as mentioned above, wherein the *ychE* gene is a DNA selected from the group consisting of (a) and (b) mentioned below:
   (a) a gene having the nucleotide sequence of SEQ ID NO: 12 or 14;
   (b) a DNA that hybridizes with a sequence complementary to the nucleotide sequence of SEQ ID NO: 12 or 14 or a probe that can be prepared from the nucleotide sequence under stringent conditions, and codes for a protein having an activity of improving L-cysteine-producing ability of the *Enterobacteriaceae* bacterium compared with a non-modified strain when expression thereof is increased in the bacterium.
[8] The method as mentioned above, wherein expression of the gene(s) is increased by increasing the copy number of the gene(s), or by modifying an expression control sequence of the gene(s).
[9] The method as mentioned above, wherein the bacterium is a bacterium belonging to the genus *Pantoea*.
[10] The method as mentioned above, wherein the bacterium is *Pantoea ananatis.*
[11] The method as mentioned above, wherein the bacterium is a bacterium belonging to the genus *Escherichia.*
[12] The method as mentioned above, wherein the bacterium is *Escherichia coli.*
[13] The method as mentioned above, wherein the related substance is L-cystine or a thiazolidine derivative.
[14] The method as mentioned above, wherein the related substance is O-acetylserine.

### Modes for Carrying out the Invention

### <1> Bacterium used in the method of present invention

The bacterium used in the method of the present invention (henceforth also referred to as "bacterium of the present invention") is a bacterium belonging to the family *Enterobacteriaceae,* which has L-cysteine-producing ability and is modified so that expression of one or more genes selected from the group consisting of the *ygaZH* genes and the *ychE* gene is increased.

In the present invention, L-cysteine can be L-cysteine in the free form, a salt thereof, or a mixture thereof. In the present invention, O-acetylserine can be O-acetylserine in the free form, a salt thereof, or a mixture thereof. The same shall apply to the other L-cysteine related substances mentioned later. Examples of the salt include, for example, sulfate, hydrochloride, carbonate, ammonium salt, sodium salt, and potassium salt.

In the present invention, the L-cysteine-producing ability refers to an ability of the bacterium of the present invention to produce L-cysteine, a related substance thereof, or a mixture thereof and accumulate it in a medium or cells of the bacterium in such an amount that L-cysteine, the related substance thereof, or the mixture thereof can be collected from the medium or cells, when the bacterium is cultured in the medium. A bacterium having L-cysteine-producing ability means a bacterium that can produce and accumulate L-cysteine, a related substance thereof, or a mixture thereof in a medium or cells in a larger amount compared with a wild-type strain or a parent strain and preferably means a bacterium that can produce and accumulate L-cysteine, a related substance thereof, or a mixture thereof in a medium in an amount of 0.2 g/L or more, more preferably 0.3 g/L or more, particularly preferably 0.4 g/L or more.

A portion of L-cysteine produced by the bacterium can be converted into L-cystine in the medium by formation of a disulfide bond. Further, S-sulfocysteine can be generated by the reaction of L-cysteine and thiosulfate contained in the medium (Szczepkowski T.W., Nature, vol. 182 (1958)). Furthermore, L-cysteine generated in bacterial cells can be condensed with a ketone or aldehyde, for example, pyruvic acid, which exists in the cells, to produce a thiazolidine derivative via a hemithioketal as an intermediate (Japanese Patent No. 2992010). These thiazolidine derivative and hemithioketal can exist as an equilibrated mixture. Furthermore, when the bacterium of the present invention is cultured in a medium, a precursor of L-cysteine can be generated. Examples of the precursor of L-cysteine include O-acetylserine and an isomer thereof, N-acetylserine. Therefore, the L-cysteine-producing ability is not limited to an ability to accumulate only L-cysteine in a medium or cells, but also includes an ability to accumulate L-cysteine, L-cystine, such a derivative of L-cysteine as mentioned above (for example, S-sulfocysteine, a thiazolidine derivative, and a hemithioketal), such a precursor of L-cysteine as mentioned above, or a mixture thereof in the medium. In the present invention, L-cystine, such a derivative of L-cysteine as mentioned above, and such a precursor of L-cysteine as mentioned above are collectively referred to as "related substance of L-cysteine".

The bacterium having L-cysteine-producing ability can be a bacterium inherently having the L-cysteine-producing ability, or it can be a bacterium obtained by modifying a bacterium such as those described below by mutagenesis or a recombinant DNA technique so that it acquires L-cysteine-producing ability.

The bacterium used for the present invention is not particularly limited, so long as a bacterium belonging to the family *Enterobacteriaceae* such as those of the genera *Escherichia, Enterobacter, Pantoea, Klebsiella, Serratia, Erwinia, Salmonella,* and *Morganella* and having L-cysteine-producing ability is chosen. Specifically, those classified into the family *Enterobacteriaceae* according to the taxonomy used in the NCBI (National Center for Biotechnology Information) database (http://www.ncbi.nlm.nih.gov/Taxonomy/Browser/wwwtax.cgi?id 91347) can be used. As a parent strain of the bacterium belonging to the family *Enterobacteriaceae* used for the modification, it is desirable to use, especially, a bacterium of the genus *Escherichia, Enterobacter, Pantoea, Erwinia,* or *Klebsiella.*

Although the *Escherichia* bacteria are not particularly limited, specifically, those described in the work of Neidhardt et al. (Backmann B.J., 1996, Derivations and Genotypes of some mutant derivatives of Escherichia coli K-12, p.2460-2488, Table 1, In F.D. Neidhardt (ed.), Escherichia coli and Salmonella Cellular and Molecular Biology/Second Edition, American Society for Microbiology Press, Washington, D.C.) can be used. Examples thereof include, for example, *Escherichia coli.* Specific examples of *Escherichia coli* include the *Escherichia coli* W3110 strain (ATCC 27325), *Escherichia coli* MG1655 strain (ATCC 47076) and so forth derived from the prototype wild-type strain, K12 strain.

These strains are available from, for example, the American Type Culture Collection (Address: 12301 Parklawn Drive, Rockville, Maryland 20852, P.O. Box 1549, Manassas, VA 20108, United States of America). That is, registration numbers are given to each of the strains, and the strains can be ordered by using these registration numbers (refer to http://www.atcc.org/). The registration numbers of the strains are listed in the catalogue of the American Type Culture Collection.

Examples of the *Enterobacter* bacteria include *Enterobacter agglomerans* and *Enterobacter aerogenes.* An example of typical strains of the Enterobacter bacteria includes *Enterobacter agglomerans* ATCC 12287 strain. Also, specifically, the strains exemplified in European Patent Publication No. 952221 can be used.

Examples of the Pantoea bacteria include *Pantoea ananatis, Pantoea stewartii, Pantoea agglomerans,* and *Pantoea citrea.*

Specific examples of *Pantoea ananatis* include the *Pantoea ananatis* AJ13355 strain (FERM BP-6614), SC17 strain (FERM BP-11091), and SC17(0) strain (VKPM B-9246). The AJ13355 strain is a strain isolated from soil in Iwata-shi, Shizuoka-ken, Japan as a strain that can proliferate in a low pH medium containing L-glutamic acid and a carbon source. The SC17 strain is a strain selected as a low phlegm-producing mutant strain from the AJ13355 strain (U.S. Patent No. 6,596,517). The SC17(0) strain was constructed as a strain resistant to the λ Red gene products for performing gene disruption in *Pantoea ananatis* (WO2008/075483).

The SC17 strain was deposited at the National Institute of Advanced Industrial Science and Technology, International Patent Organism Depository (address: Tsukuba Central 6, 1-1, Higashi 1-Chome, Tsukuba-shi, Ibaraki-ken, 305-8566, Japan) on February 4, 2009, and assigned an accession number of FERM ABP-11091. The SC17(0) strain was deposited at the Russian National Collection of Industrial Microorganisms (VKPM), GNII Genetika (address: Russia, 117545 Moscow, 1 Dorozhny proezd. 1) on September 21, 2005 with an accession number of VKPM B-9246. The AJ13355 strain was deposited at the National Institute of Bioscience and Human-Technology, Agency of Industrial Science and Technology, Ministry of International Trade and Industry (currently, the National Institute of Advanced Industrial Science and Technology, International Patent Organism Depositary, Address: Tsukuba Central 6, 1-1, Higashi 1-Chome, Tsukuba-shi, Ibaraki-ken, 305-8566, Japan) on February 19, 1998 and assigned an accession number of FERM P-16644. It was then converted to an international deposit under the provisions of Budapest Treaty on January 11, 1999 and assigned an accession number of FERM BP-6614. The AJ13355 strain was identified as *Enterobacter agglomerans* when it was isolated, and deposited as the *Enterobacter agglomerans* AJ13355 strain. However, it was recently reclassified into *Pantoea ananatis* on the basis of nucleotide sequence analysis of 16S rRNA and so forth.

Examples of the *Erwinia* bacteria include *Erwinia amylovora* and *Erwinia carotovora,* and examples of the *Klebsiella* bacteria include *Klebsiella planticola.*

In particular, Pantoea bacteria, Erwinia bacteria, and *Enterobacter* bacteria are classified as γ-proteobacteria, and they are taxonomically very close to one another (J. Gen. Appl. Microbiol., 1997, 43, 355-361; International Journal of Systematic Bacteriology, Oct. 1997, pp.1061-1067). Some bacteria belonging to the genus *Enterobacter* were recently reclassified into *Pantoea agglomerans, Pantoea dispersa,* or the like, on the basis of DNA-DNA hybridization experiments etc. (International Journal of Systematic Bacteriology, July 1989, 39(3), pp.337-345). For example, some strains of *Enterobacter agglomerans* were reclassified into *Pantoea agglomerans, Pantoea ananatis,* or *Pantoea stewartii* on the basis of nucleotide sequence analysis of 16S rRNA etc. Furthermore, some bacteria belonging to the genus *Erwinia* were reclassified into Pantoea *ananas* or *Pantoea stewartii* (refer to International Journal of Systematic Bacteriology, Jan 1993;43(1), pp.162-173). The bacterium of the present invention can be a bacterium belonging to any of the genus *Enterobacter, Pantoea, Erwinia,* and the like so long as it is a bacterium classified into the family *Enterobacteriaceae.*

### [Methods for imparting or enhancing L-cysteine-producing ability, and L-cysteine-producing bacteria]

To impart L-cysteine-producing ability to a bacterium, methods conventionally employed in the breeding of coryneform bacteria, *Escherichia* bacteria, and so forth can be used. Such methods include acquiring an auxotrophic mutant strain, an analogue resistant strain, or a metabolic regulation mutant strain, or constructing a recombinant strain in which an L-cysteine biosynthesis enzyme is overexpressed, and so forth (see "Amino Acid Fermentation", Gakkai Shuppan Center (Ltd.), 1st Edition, published May 30, 1986, pp.77-100). In the breeding of L-cysteine-producing bacteria, one or two or more kinds of the above-described properties such as auxotrophy, analogue resistance, and metabolic regulation mutation can be imparted. Also, expression of one or two or more kinds of the L-cysteine biosynthesis enzymes can be enhanced. Furthermore, imparting such properties as auxotrophy, analogue resistance, and metabolic regulation mutation can be combined with enhancing a biosynthesis enzyme.

Methods for imparting L-cysteine-producing ability to bacteria belonging to the family *Enterobacteriaceae* or enhancing L-cysteine-producing ability of such bacteria, and bacteria having L-cysteine-producing ability are specifically exemplified below.

An auxotrophic mutant strain, L-cysteine analogue resistant strain, or metabolic regulation mutant strain having L-cysteine-producing ability can be obtained by subjecting a parent strain or wild-type strain to conventional mutagenesis, such as exposure to X-rays or UV irradiation or a treatment with a mutagen such as N-methyl-N'-nitro-N-nitrosoguanidine (MNNG) or ethyl methanesulfonate (EMS), and then selecting a strain exhibiting autotrophy, analogue resistance or a metabolic regulation mutation and having L-cysteine-producing ability from the obtained mutant strains.

L-cysteine-producing ability can be imparted to a bacterium or L-cysteine-producing ability of a bacterium can be enhanced by enhancing the L-cysteine biosynthesis system. "To enhance the L-cysteine biosynthesis system" means, for example, to enhance the activity/activities of enzyme(s) involved in the biosynthesis of L-cysteine. Examples of the enzyme involved in the biosynthesis of L-cysteine include, for example, enzymes of the L-cysteine biosynthesis pathway, and enzymes involved in synthesis of a compound serving as a substrate in the L-cysteine biosynthesis pathway, such as L-serine. Specific examples of such enzymes include serine acetyltransferase (SAT) and 3-phosphoglycerate dehydrogenase (PGD). Enhancement of the activity of an enzyme can be attained by enhancing expression of a gene coding for the objective enzyme or by enhancing specific activity of the objective enzyme, as described later.

For example, expression of a gene coding for SAT can be enhanced by introducing a gene coding for SAT into an appropriate vector and then introducing the vector into a bacterium, or by introducing a gene coding for SAT into the chromosome of a bacterium. As the gene coding for SAT, any of genes derived from *Escherichia* bacteria and genes derived from other organisms can be used. As the gene coding for SAT of *Escherichia coli, cycE* has been cloned from a wild-type strain and an L-cysteine secretion mutant strain, and the nucleotide sequence thereof has been elucidated (Denk, D. and Boeck, A., J. General Microbiol., 133, 515-525 (1987)). Therefore, a gene coding for SAT can be obtained by PCR utilizing primers prepared on the basis of the nucleotide sequence thereof (SEQ ID NO: 16) and chromosomal DNA of an *Escherichia* bacterium as the template (refer to Japanese Patent Laid-open (Kokai) No. 11-155571). Genes coding for SAT of other organisms can also be obtained in a similar manner.

Furthermore, because serine acetyltransferase is inhibited by feedback inhibition by L-cysteine, the enzymatic activity of this enzyme can be enhanced by, in particular, incorporating a mutant type *cysE* gene coding for serine acetyltransferase of which feedback inhibition is attenuated or eliminated into a bacterium. Furthermore, because 3-phosphoglycerate dehydrogenase is inhibited by feedback inhibition by serine, the enzymatic activity of this enzyme can be enhanced by, in particular, incorporating a mutant type *serA* gene coding for a mutant 3-phosphoglycerate dehydrogenase of which feedback inhibition is attenuated or eliminated into a bacterium.

In the present invention, SAT of which feedback inhibition is attenuated or eliminated is also referred to as "mutant SAT". Examples of the mutant SAT include SAT having a mutation replacing an amino acid residue corresponding to the methionine residue at position 256 of the wild-type SAT of *Escherichia coli* (SEQ ID NO: 17) with an amino acid residue other than lysine residue and leucine residue, or a mutation deleting a C-terminus side region from an amino acid residue corresponding to the methionine residue at position 256 of the wild-type SAT of *Escherichia* coli (Japanese Patent Laid-open (Kokai) No. 11-155571). Examples of the amino acid residue other than lysine residue and leucine residue include the 17 kinds of amino acid residues among the general amino acid residues constituting proteins except for methionine, lysine, and leucine residues. Preferred examples of the amino acid residue other than lysine residue and leucine residue include isoleucine residue and glutamic acid residue. As mutant SAT, there are also known the mutant SAT having one or more mutations in the amino acid sequence corresponding to positions 89 to 96 of the wild-type SAT (U.S. Patent Published Application No. 20050112731(A1)), the mutant SAT in which the valine residue and the aspartic acid residue at positions 95 and 96 of the wild-type SAT are replaced with arginine residue and proline residue, respectively (name of the mutant gene, *cysE5;* U.S. Patent Published Application No. 20050112731(A1)), the mutant SAT in which the threonine residue at position 167 of the wild-type SAT is replaced with alanine residue (U.S. Patent No. 6,218,168, U.S. Patent Published Application No. 20050112731(A1)), and so forth. Examples of the method for introducing a desired mutation into a wild-type SAT gene include site-specific mutagenesis method, overlap extension method, and so forth. Although the codon of the mutated amino acid residue is not particularly limited, so long as it codes for the intended amino acid, it is preferable to use a codon frequently used in the objective Enterobacteriaceae bacterium. The *Escherichia coli* JM39-8 strain harboring a plasmid pCEM256E containing a mutant cysE coding for the mutant SAT in which the methionine residue at position 256 is replaced with glutamic acid residue (*E. coli* JM39-8(pCEM256E); private number, AJ13391) was deposited at the National Institute of Bioscience and Human-Technology, Agency of Industrial Science and Technology, Ministry of International Trade and Industry (currently, the National Institute of Advanced Industrial Science and Technology, International Patent Organism Depositary, Address: Tsukuba Central 6, 1-1, Higashi 1-Chome, Tsukuba-shi, Ibaraki-ken, 305-8566, Japan) on November 20, 1997, and assigned an accession number of FERM P-16527. The deposit was then converted to an international deposit under the provisions of Budapest Treaty on July 8, 2002, and assigned an accession number of FERM BP-8112.

Although the mutant SAT can be SAT which has been modified so that it is desensitized to feedback inhibition by L-cysteine as described above, it can also be SAT inherently free from feedback inhibition by L-cysteine. For example, SAT of *Arabidopsis thaliana* is known to be free from feedback inhibition by L-cysteine, and can be suitably used for the present invention. As a plasmid containing the SAT gene derived from *Arabidopsis thaliana,* pEAS-m is known (FEMS Microbiol. Lett., 179 (1999) 453-459).

Furthermore, as a gene coding for a mutant PGD resistant to feedback inhibition by serine, the serA5 gene (U.S. Patent No. 6,180,373) is known.

Furthermore, L-cysteine-producing ability of a bacterium can also be improved by enhancing the L-cysteine secretion system. The L-cysteine secretion system can be enhanced by enhancing the activity of a protein involved in secretion of L-cysteine. As proteins involved in secretion of L-cysteine, there are known the protein encoded by the *ydeD* gene (Japanese Patent Laid-open (Kokai) No. 2002-233384), the protein encoded by the yfiK gene (Japanese Patent Laid-open (Kokai) No. 2004-49237), the respective proteins encoded by the *emrAB, emrKY, yojIH, acrEF, bcr,* and cusA genes (Japanese Patent Laid-open (Kokai) No. 2005-287333), and the protein encoded by the yeaS gene (Japanese Patent Laid-open (Kokai) No. 2010-187552). Therefore, by enhancing expression of one or more genes selected from these genes, the L-cysteine secretion system can be enhanced, and thereby L-cysteine-producing ability can be enhanced. Furthermore, by introducing a mutation into the threonine residue of position 28, the phenylalanine residue of position 137, and/or the leucine residue of position 188 of the YeaS protein, the L-cysteine secretion system is also enhanced, and thereby L-cysteine-producing ability can be enhanced (European Patent Laid-open No. 2218729). Specifically, a mutation for replacing the threonine residue at position 28 with asparagine residue, a mutation for replacing the phenylalanine residue at position 137 with serine, glutamine, alanine, histidine, cysteine, or glycine residue, and/or a mutation for replacing the leucine residue at position 188 with glutamine residue in the YeaS protein is preferred (European Patent Laid-open No. 2218729).

Furthermore, L-cysteine-producing ability can also be increased by enhancing expression of gene(s) of the *cysPTWAM* cluster coding for the sulfate/thiosulfate transport system proteins (Japanese Patent Laid-open (Kokai) No. 2005-137369, European Patent Laid-open No. 1528108).

Furthermore, sulfide is incorporated into O-acetyl-L-serine via the reaction catalyzed by O-acetylserine (thiol) lyases A and B encoded by the *cysK* gene and the *cysM gene,* respectively, and then L-cysteine is produced. Therefore, these enzymes are included in the enzymes of the L-cysteine biosynthesis pathway, and hence, L-cysteine-producing ability can also be enhanced by enhancing expression of gene(s) coding for these enzyme(s).

Moreover, L-cysteine-producing ability of a bacterium can also be improved by suppressing the L-cysteine decomposition system. "To suppress the L-cysteine decomposition system" means that the intracellular L-cysteine decomposition activity is decreased compared with that of a non-modified strain such as a wild-type strain or parent strain. As proteins responsible for the L-cysteine decomposition, cystathionine-β-lyase encoded by the *metC* gene (Japanese Patent Laid-open (Kokai) No. 11-155571, Chandra et al., Biochemistry, 21, 3064-3069 (1982)), tryptophanase encoded by the *tnaA* gene (Japanese Patent Laid-open (Kokai) No. 2003-169668, Austin Newton et al., J. Biol. Chem., 240, 1211-1218 (1965)), O-acetylserine sulfhydrylase B encoded by the *cysM* gene (*cysM gene* product; Japanese Patent Laid-open (Kokai) No. 2005-245311), and MalY encoded by the *malY* gene (Japanese Patent Laid-open (Kokai) No. 2005-245311) are known. Furthermore, as a gene coding for a protein having the cysteine desulfhydrase activity, the *d0191* gene of *Pantoea ananatis* is known (Japanese Patent Laid-open (Kokai) No. 2009-232844). By decreasing the activity/activities of these protein(s), L-cysteine-producing ability can be improved. The enzyme activity can be decreased by the methods described later.

Furthermore, in the present invention, in particular, the ability to accumulate O-acetylserine in a medium or cells can be referred to as O-acetylserine-producing ability. O-acetylserine is easily converted into N-acetylserine by a spontaneous reaction in a neutral to alkaline pH region. However, for example, by maintaining pH of culture medium to be in the acidic range, such conversion into N-acetylserine can be prevented (U.S. Patent Published Application No. 20020146783(A1)). Since such a technique can be used together in the production of O-acetylserine by fermentation, it can be considered that O-acetylserine-producing ability also includes an ability to accumulate N-acetylserine in a medium.

O-acetylserine is a precursor of L-cysteine, and therefore, O-acetylserine and L-cysteine share a common biosynthetic pathway. Therefore, O-acetylserine-producing ability can be imparted to a bacterium or O-acetylserine-producing ability of a bacterium can be enhanced by enhancing the L-cysteine biosynthesis pathway. That is, for example, use of a mutant serine acetyltransferase of which feedback inhibition by cysteine is attenuated or eliminated or a mutant 3-phosphoglycerate dehydrogenase of which feedback inhibition by serine is attenuated or eliminated, or any other modification for enhancing the L-cysteine biosynthesis pathway is effective for the production of O-acetylserine.

O-acetylserine reacts with a sulfur source thereby to be converted into L-cysteine. Therefore, in order to attain high production of O-acetylserine, it is rather preferable not to enhance the O-acetylserine (thiol) lyases A and B, which are encoded by the *cysK* gene and the *cysM* gene, respectively, and catalyze the reaction of converting O-acetylserine into L-cysteine, or the pathway involved in supply of the sulfur source, which is a substrate of that reaction, so that the reaction converting O-acetylserine into L-cysteine should not unduly proceed. Furthermore, in order to suppress the reaction converting O-acetylserine into L-cysteine, supply of the sulfur source as a substrate of the reaction can be restricted. For example, by restricting addition of thiosulfate in the fermentation in which thiosulfate is added as a sulfur source, or by restricting addition of sulfate in the fermentation in which sulfate is added as a sulfur source, O-acetylserine can be efficiently produced.

O-acetylserine-producing ability can also be improved by enhancing O-acetylserine-secreting ability. It is known that some of L-cysteine secretion factors such as mentioned above are also involved in secretion of O-acetylserine. Therefore, for example, by enhancing expression of the *ydeD* gene coding for the YdeD protein (Dassler et al., Mol. Microbiol., 36, 1101-1112 (2000)) or the *yfiK gene* coding for the YfiK protein (Franke I. et al., J. Bacteriol., 2003 Feb, 185(4):1161-6), O-acetylserine-producing ability can be improved.

The L-cysteine-producing bacterium can have any one of the characteristics for improving L-cysteine-producing ability such as mentioned above solely, or two or more of them in an arbitrary combination. The L-cysteine-producing bacterium used in the present invention preferably has at least enhanced L-cysteine biosynthesis system, more preferably a mutant SAT, among the characteristics for improving L-cysteine-producing ability such as mentioned above.

Specific examples of L-cysteine-producing bacteria or a parent strain for deriving it include, but not limited to, *Escherichia* bacteria such as the *E. coli* JM15 transformed with multiple kinds of *cysE* gene alleles encoding serine acetyltransferase (SAT) resistant to feedback inhibition (U.S. Patent 6,218,168), *E. coli* W3110 in which a gene encoding a protein suitable for excreting a cytotoxic substance is enhanced (U.S. Patent No. 5,972,663), *E. coli* in which cysteine desulfhydrase activity is decreased (Japanese Patent Laid-open (Kokai) No. 11-155571), *E. coli* W3110 in which activity of the positive transcriptional control factor of the cysteine regulon encoded by the *cysB* gene is increased (WO01/27307), and *E. coli* having the plasmid pACYC-DES (Japanese Patent Laid-open (Kokai) No. 2005-137369 (U.S. Patent Published Application No. 20050124049(A1), European Patent Laid-open No. 1528108(A1))) containing the *ydeD* gene, a mutant *cysE* gene, and a mutant *serA5* gene. pACYC-DES is a plasmid obtained by inserting the above three kinds of genes into pACYC184, and expression of each of the genes is controlled by the ompA promoter (PompA).

Methods for increasing the activity of a protein such as SAT and for reducing the activity of a protein such as cysteine desulfhydrase are exemplified below.

### <Method for increasing activity of protein>

In the present invention, the expression "activity of a protein is increased" means that the activity of an objective protein is increased compared with that of a non-modified strain such as a wild-type strain or a parent strain. Although the degree of the increase of the activity of the protein is not particularly limited so long as the activity is increased compared with a non-modified strain, the activity is increased preferably 1.5 times or more, more preferably 2 times or more, still more preferably 3 times or more, compared with that of a non-modified strain. Further, the case where "activity of a protein is increased" includes not only a case where the activity of an objective protein is increased in a strain natively having the activity of the protein, but also a case where the activity of an objective protein is imparted to a strain not natively having the activity of the protein.

A modification for increasing the activity of a protein can be attained by, for example, enhancing expression of a gene coding for the protein. In the present invention, the expression "enhancement of expression of a gene" can be used for the same meaning as "increase of expression of a gene".

Enhancement of expression of a gene can be attained by, for example, increasing the copy number of the gene.

The copy number of an objective gene can be increased by introducing the gene into the chromosome of a host microorganism. The objective gene can be introduced into the chromosome by a method of randomly introducing a gene into the chromosome using a transposon or Mini-Mu (Japanese Patent Laid-open (Kokai) No. 2-109985, U.S. Patent No. 5,882,888, European Patent Publication No. 805867 B1), or by homologous recombination using a sequence present on the chromosomal DNA in a multiple copy number as a target. As a sequence present on the chromosomal DNA in a multiple copy number, a repetitive DNA, and inverted repeats located at the both ends of a transposon can be used. Alternatively, an objective gene can also be introduced into the chromosome by using the Red driven integration method (WO2005/010175). Moreover, an objective gene can also be introduced into the chromosome by transduction using a phage such as P1 phage, or by using a conjugative transfer vector. Furthermore, an objective gene can also be introduced by using a gene unnecessary for production of an objective substance as a target, as described in WO03/040373. One or plural copies of the objective gene can be introduced into a target sequence by such methods as described above.

Introduction of an objective gene on the chromosome can be confirmed by Southern hybridization using a probe having a sequence complementary to a part or the whole of the objective gene, PCR using primers prepared on the basis of the sequence of the objective gene, or the like.

Further, the copy number of an objective gene can also be increased by introducing a vector containing the gene into a host bacterium. For example, the copy number of an objective gene can be increased by ligating a DNA fragment containing the objective gene with a vector that functions in a host bacterium to construct an expression vector of the objective gene, and transforming the host bacterium with the expression vector. As the vector, a vector autonomously replicable in the cell of the host bacterium can be used. The vector is preferably a multicopy vector. Further, the vector preferably comprises a marker such as an antibiotic resistance gene for selection of the transformants. Examples of plasmid autonomously replicable in a microorganism belonging to the family *Enterobacteriaceae* include pUC19, pUC18, pBR322, RSF1010, pHSG299, pHSG298, pHSG399, pHSG398, pSTV28, pSTV29, pTWV228, pTWV229 (pHSG, pSTV, and pTWV series vectors are available from Takara Bio), pMW119, pMW118, pMW219, pMW218 (pMW series vectors are available from Nippon Gene), pTrc99A (Pharmacia), pPROK series vectors (Clontech), pKK233-2 (Clontech), pET series vectors (Novagen), pQE series vectors (QIAGEN), a broad host-range vector RSF1010, and so forth.

Further, expression of a gene can be enhanced by improving the transcription efficiency of the gene. The transcription efficiency of a gene can be improved by, for example, substituting a stronger promoter for the promoter of the gene on the chromosome. The "stronger promoter" means a promoter providing improved transcription of a gene compared with the natively existing wild-type promoter. As a stronger promoter, for example, known high expression promoters, such as T7 promoter, trp promoter, lac promoter, tac promoter, and PL promoter, can be used. Further, as the stronger promoter, a highly-active type of a usual promoter can be obtained by using various reporter genes. For example, by making the -35 and -10 regions in a promoter region closer to the consensus sequence, the activity of the promoter can be enhanced (International Patent Publication WO00/18935). Methods for evaluating the strength of promoters and examples of strong promoter are described in the paper of Goldstein et al. (Prokaryotic Promoters in Biotechnology, Biotechnol. Annu. Rev., 1, 105-128 (1995)), and so forth.

Further, expression of a gene can also be enhanced by improving the translation efficiency of the gene. The translation efficiency of a gene can be improved by, for example, replacing the Shine-Dargarno (SD) sequence (also referred to as ribosome binding site (RBS)) on the chromosome with a stronger SD sequence. The "stronger SD sequence" means a SD sequence that provides improved translation of mRNA compared with the natively existing wild-type SD sequence. Examples of the stronger SD sequence include, for example, RBS of the gene 10 derived from phage T7 (Olins P.O. et al, Gene, 1988, 73, 227-235). Furthermore, it is known that substitution, insertion, or deletion of several nucleotides in a spacer region between RBS and the start codon, especially in a sequence immediately upstream of the start codon (5'-UTR), significantly affects the stability and translation efficiency of mRNA, and the translation efficiency of a gene can also be improved by modifying them.

In the present invention, sites affecting gene expression, such as promoter, SD sequence, and spacer region between RBS and the start codon, are also collectively called "expression control regions" or "expression control sequences". An expression control region can be determined by using a promoter-search vector or gene analysis software such as GENETYX. Such expression control regions can be modified by, for example, a method using a temperature sensitive vector or the Red driven integration method (WO2005/010175).

Furthermore, expression of an objective gene can also be enhanced by amplifying a regulator that increases expression of the gene, or deleting or attenuating a regulator that reduces expression of the gene. As regulators that reduce expression of an objective gene include, there are known the proteins belonging to the Lrp family (Kutukova et al., FEBS Lett., 579, 4629-4634 (2005)), and so forth. Such regulators can also be found by using a database, such as EcoCyc (http://ecocyc.org/). A regulator can be modified with monitoring increase of the activity of an objective protein, increase of the transcription amount of an objective gene, or increase of the amount of the objective protein as an index.

Such methods for enhancing gene expression as mentioned above can be used independently or in an arbitrary combination.

Further, a modification that increases the activity of a protein can also be attained by, for example, enhancing the specific activity of the objective protein. A protein showing an enhanced specific activity can be obtained by, for example, searching various bacteria. Further, a highly-active type of a usual protein can also be obtained by introducing a mutation into the usual protein. Enhancement of the specific activity can be independently used, or can be used in an arbitrary combination with such methods for enhancing gene expression as mentioned above.

The method of the transformation is not particularly limited, and conventionally known methods can be used. There can be used, for example, methods of treating recipient cells with calcium chloride so as to increase permeability thereof for DNA, which has been reported for *Escherichia coli* K-12 strain (Mandel, M. and Higa, A., J. Mol. Biol., 53:159-162 (1970)), and preparing competent cells from cells which are at the growth phase, followed by transformation with DNA, which has been reported for *Bacillus subtilis* (Duncan, C.H., Wilson, G.A. and Young, F.E., Gene, 1, 153 (1977)). Alternatively, there can also be used a method of making DNA-recipient cells into protoplasts or spheroplasts, which can easily take up recombinant DNA, followed by introducing a recombinant DNA into the cells, which is known to be applicable to *Bacillus subtilis,* actinomycetes and yeasts (Chang, S. and Choen, S.N., 1979, Mol. Gen. Genet., 168:111-115; Bibb, M.J., Ward, J.M. and Hopwood, O.A., 1978, Nature, 274:398-400; Hinnen, A., Hicks, J.B. and Fink, G.R., 1978, Proc. Natl. Acad. Sci. USA, 75:1929-1933). Furthermore, a microorganism can also be transformed by the electric pulse method (Japanese Patent Laid-open (Kokai) No. 2-207791).

Increase of the activity of an objective protein can be confirmed by measuring the activity of the protein.

Increase of the expression of an objective gene can be confirmed by confirming increase of the transcription amount of the objective gene, or by confirming increase of the amount of the objective protein.

Increase of the transcription amount of an objective gene can be confirmed by comparing amount of mRNA transcribed from the gene with that of a non-modified strain such as a wild strain or a parent strain. Examples of method for evaluating the amount of mRNA include Northern hybridization, RT-PCR, and so forth (Sambrook, J., et al., Molecular Cloning A Laboratory Manual/Third Edition, Cold spring Harbor Laboratory Press, Cold Spring Harbor (USA), 2001). The amount of mRNA preferably increases, for example, 1.5 times or more, 2 times or more, or 3 times or more, compared with that of a non-modified strain.

Increase of the amount of an objective protein can be confirmed by Western blotting using an antibody (Molecular Cloning, Cold Spring Harbor Laboratory Press, Cold Spring Harbor (USA), 2001). The amount of the objective protein preferably increases, for example, 1.5 times or more, 2 times or more, or 3 times or more, compared with that of a non-modified strain.

### <Method for reducing activity of protein>

In the present invention, the expression "activity of a protein is reduced" means that the activity of an objective protein is decreased compared with that of a non-modified strain such as a wild-type strain or a parent strain, and includes a case where the activity has completely disappeared. Although the degree of the reduction of the activity of the protein is not particularly limited so long as the activity is reduced compared with that of a non-modified strain, the activity is preferably reduced to, for example, 75% or less, 50% or less, 25% or less, or 10% or less compared with that of a non-modified strain, and the complete disappearance of the activity is particularly preferred. Depending on the type of protein, it can also be rather preferable not to completely eliminate the activity.

A modification for reducing the activity of a protein can be attained by, for example, reducing expression of a gene coding for the protein. Expression of a gene can be reduced by, for example, modifying an expression control sequence, such as promoter and Shine-Dargarno (SD) sequence, of the gene. When an expression control sequence is modified, one or more nucleotides, more preferably two or more nucleotides, particularly preferably three or more nucleotides, of the expression control sequence are modified. Further, a part or all of the expression control sequence can be deleted. Further, expression of a gene can also be reduced by expressing an antisense RNA.

Further, a modification for reducing the activity of a protein can also be attained by, for example, deleting a part or all of a coding region of a gene coding for the protein on the chromosome. Further, the whole gene including the sequences upstream and downstream of the gene on a chromosome can be deleted. Further, the whole gene including the sequences upstream and downstream of the gene on a chromosome can be deleted. The region to be deleted can be an N-terminus region, an internal region or a C-terminus region, so long as the activity of the protein can be reduced. Deletion of a longer region can usually more surely inactivate a gene. Furthermore, it is preferred that reading frames upstream and downstream of the region to be deleted are not the same.

Further, a modification for reducing the activity of a protein can also be attained by introducing a mutation for amino acid substitution (missense mutation), a stop codon (nonsense mutation), or a frame shift mutation which adds or deletes one or two nucleotides into a coding region of a gene coding for the protein on the chromosome (Journal of Biological Chemistry, 272:8611-8617 (1997); Proceedings of the National Academy of Sciences, USA, 95 5511-5515 (1998); Journal of Biological Chemistry, 266, 20833-20839 (1991)).

Further, a modification for reducing the activity of a protein can also be attained by inserting another sequence into a coding region of a gene coding for the protein on the chromosome. Such a sequence can be inserted into any region of the gene, and insertion of a longer sequence can more surely inactivate the objective gene. It is preferred that reading frames upstream and downstream of the insertion site are not the same. The other sequence is not particularly limited so long as a sequence which decreases or deletes the activity of the encoded protein is chosen, and examples include a marker gene such as antibiotic resistance genes, a gene useful for L-cysteine production, and so forth.

A gene on the chromosome can be modified as described above by, for example, preparing a deletion-type gene of the gene, in which a partial sequence of the gene is deleted so that the deletion-type gene does not produce a protein that can normally function, and then transforming a bacterium with a recombinant DNA containing the deletion-type gene to cause homologous recombination between the deletion-type gene and the native gene on the chromosome, which results in substitution of the deletion-type gene for the native gene on the chromosome. The above operation can be made easier by introducing a marker gene suitable for a characteristic of the host such as auxotrophy into the recombinant DNA. The protein encoded by the deletion-type gene has a conformation different from that of the wild-type protein, even if it is even produced, and thus the function is reduced or deleted. Such gene disruption based on gene substitution utilizing homologous recombination has already been established, and examples include the method called Red-driven integration (Datsenko, K.A., and Wanner, B.L., Proc. Natl. Acad. Sci. USA, 97:6640-6645 (2000)), methods using a linear DNA such as the method of utilizing Red driven integration in combination with an excision system derived from λ phage (Cho, E.H., Gumport, R.I., Gardner, J.F., J. Bacteriol., 184:5200-5203 (2002)) (refer to WO2005/010175), methods using a plasmid containing a temperature sensitive replication origin, methods using a plasmid capable of conjugative transfer, methods utilizing a suicide vector without a replication origin in a host (U.S. Patent No. 6,303,383, Japanese Patent Laid-open (Kokai) No. 05-007491), and so forth.

Further, a modification for reducing the activity of a protein can also be attained by, for example, mutagenesis. Examples of mutagenesis include usual mutagenesis treatments such as exposure to X-ray or UV irradiation or a treatment with a mutagen such as N-methyl-N'-nitro-N-nitrosoguanidine (MNNG), ethyl methanesulfonate (EMS), and methyl methanesulfonate (MMS).

Decrease of the activity of an objective protein can be confirmed by measuring the activity of the protein. The cysteine desulfhydrase activity can be measured by the method described in Japanese Patent Laid-open (Kokai) No. 2002-233384.

Decrease of the expression of an objective gene can be confirmed by confirming decrease of the transcription amount of the gene or decrease of the amount of the objective protein expressed from the gene.

Decrease of the transcription amount of an objective gene can be confirmed by comparing the amount of mRNA transcribed from the gene with that of a non-modified strain. Examples of method for evaluating amount of mRNA include Northern hybridization, RT-PCR, and so forth (Molecular Cloning, Cold Spring Harbor Laboratory Press, Cold Spring Harbor, USA, 2001). The amount of mRNA preferably decreases to, for example, 50% or less, 20% or less, 10% or less, 5% or less, or 0%, of that observed in a non-modified strain.

Decrease of the amount of an objective protein can be confirmed by Western blotting using an antibody (Molecular Cloning, Cold Spring Harbor Laboratory Press, Cold Spring Harbor, USA, 2001). The amount of an objective protein preferably decreases to, for example, 50% or less, 20% or less, 10% or less, 5% or less, or 0%, of that observed in a non-modified strain.

### [ygaZH genes and ychE gene]

The bacterium of the present invention can be obtained by modifying such a bacterium belonging to the family *Enterobacteriaceae* and having L-cysteine-producing ability as mentioned above so that expression of one or more genes selected from the *ygaZH* genes and the *ychE* gene increases. The bacterium of the present invention can also be obtained by modifying a bacterium belonging to the family *Enterobacteriaceae* so that expression of one or more genes selected from the *ygaZH* genes and the *ychE* gene increases, and then imparting L-cysteine-producing ability to the bacterium.

The *ygaZH* genes and the *ychE* gene will be explained below.

The *ygaZ* gene of the *Escherichia coli* K12 MG1655 strain corresponds to the sequence of positions 2807639 to 2808376 in the genome sequence registered at the NCBI database as GenBank accession NC_000913 (VERSION NC_000913.2 GI: 49175990). The ygaZ gene of the *Escherichia coli* K12 MG1655 strain is the same as ECK2676 and JW2657. The *YgaZ* protein of the *Escherichia coli* K12 MG1655 strain is registered as GenBank accession NP_417167 (version NP_417167.1 GI: 16130594, locus_tag "b2682").

The *ygaH* gene of the *Escherichia coli* K12 MG1655 strain corresponds to the sequence of positions 2808366 to 2808701 in the genome sequence registered at the NCBI database as GenBank accession NC_000913 (VERSION NC_000913.2 GI: 49175990). The *ygaH* gene of the *Escherichia coli* K12 MG1655 strain is the same as ECK2677 and JW2658. The YgaH protein of the *Escherichia coli* K12 MG1655 strain is registered as GenBank accession NP_417168 (version NP_417168.1 GI: 16130595, locus_tag "b2683").

The *ychE* gene of the *Escherichia coli* K12 MG1655 strain corresponds to the sequence of positions 1297821 to 1298468 in the genome sequence registered at the NCBI database as GenBank accession NC_000913 (VERSION NC_000913.2 GI: 49175990). The ychE gene of the *Escherichia coli* K12 MG1655 strain is the same as ECK1236 and JW1229. The YchE protein of the *Escherichia coli* K12 MG1655 strain is registered as GenBank accession NP_415758 (version NP_415758.1 GI: 16129203, locus_tag "b1242").

The nucleotide sequence of the *ygaZ* gene of *Escherichia coli* and the amino acid sequence of the protein encoded by this gene are shown as SEQ ID NOS: 7 and 8, respectively. The nucleotide sequence of the *ygaH* gene of *Escherichia coli* and the amino acid sequence of the protein encoded by this gene are shown as SEQ ID NOS: 9 and 10, respectively. It is known that the *ygaZ* gene and the *ygaH* gene form an operon. The nucleotide sequence of the *ygaZH* operon of *Escherichia coli* is shown as SEQ ID NO: 11. In addition, it is known that the *YgaZ* protein and the *YgaH* protein of *Escherichia coli* form a complex and function as the complex. Therefore, when expression of the *ygaZH* genes is increased, expressions of both the *ygaZ* gene and the *ygaH* gene are increased. Expressions of the *ygaZ* gene and the *ygaH* gene can be collectively increased as the operon, or individually increased, so long as expressions of the both genes are increased as a result.

The nucleotide sequence of the *ychE* gene of *Escherichia coli* and the amino acid sequence of the protein encoded by this gene are shown as SEQ ID NOS: 12 and 13, respectively. The nucleotide sequence of the *ychE* gene of *Pantoea ananatis* and the amino acid sequence of the protein encoded by this gene are shown as SEQ ID NOS: 14 and 15, respectively.

The proteins encoded by the *ygaZH* genes and the *ychE* gene are each a protein having an activity of improving L-cysteine-producing ability of an *Enterobacteriaceae* bacterium compared with a non-modified strain when expression thereof is increased in the bacterium, and it is estimated that they each have an activity for secreting L-cysteine and O-acetylserine out of the cells.

The activity of improving L-cysteine-producing ability of an *Enterobacteriaceae* bacterium compared with a non-modified strain when expression thereof is increased in the bacterium means an activity of imparting an ability to produce L-cysteine, a related substance thereof, or a mixture thereof and accumulate it in a medium in an amount larger than that obtainable with a non-modified strain such as a wild strain or parent strain, when expression thereof is increased in the *Enterobacteriaceae* bacterium, and means an activity of imparting an ability to produce L-cysteine, a related substance thereof, or a mixture thereof and accumulate it in a medium in an amount larger than that obtainable with a non-modified strain by preferably 0.1 g/L or more, more preferably 0.2 g/L or more, particularly preferably 0.3 g/L or more.

Whether a protein has the activity of improving L-cysteine-producing ability of an *Enterobacteriaceae* bacterium compared with a non-modified strain when expression thereof is increased in the bacterium can be confirmed by preparing a strain modified so that expression of a gene of the protein is increased from the *Enterobacteriaceae* bacterium, quantifying the amount of L-cysteine, a related substance thereof, or a mixture thereof accumulated in a medium when the modified strain is cultured in the medium, and comparing this amount with the amount of L-cysteine, a related substance thereof, or a mixture thereof accumulated in a medium when the strain before the modification (non-modified strain) is cultured in the medium. As the non-modified strain referred to herein, a bacterium belonging to the family *Enterobacteriaceae* and having L-cysteine-producing ability can be used, and a specific example thereof includes a strain obtained from the *E*. *coli* MG1655 strain by enhancing a gene coding for a mutant SAT desensitized to feedback inhibition.

Furthermore, whether a protein has the activity of improving L-cysteine-producing ability of an *Enterobacteriaceae* bacterium compared with a non-modified strain when expression thereof is increased in the bacterium can also be easily confirmed by investigating O-acetylserine resistance or L-cysteine resistance. That is, if the growth of a bacterium in which expression of a gene of the protein is increased is improved in a medium containing a predetermined concentration of O-acetylserine or L-cysteine compared with a non-modified strain, it can be determined that the protein has the activity of improving L-cysteine-producing ability of an *Enterobacteriaceae* bacterium compared with a non-modified strain when expression thereof is increased in the bacterium. Specifically, it can be confirmed by inoculating a bacterium in which expression of the gene is increased into a medium containing about 0.1 to 10 mM O-acetylserine or L-cysteine, measuring diameters of colonies at an appropriate time point 10 to 120 hours after the inoculation, and confirming that these diameters are larger than the diameters of colonies of a non-modified strain cultured in a similar manner.

Since the nucleotide sequences of the *ygaZH* genes and the *ychE* gene can differ depending on the genus, species, or strain to which the bacterium belongs, the *ygaZH* genes and the *ychE* gene of which expression is increased can each be a variant of the corresponding nucleotide sequence mentioned above, so long as they each code for a protein having the activity of improving L-cysteine-producing ability of an *Enterobacteriaceae* bacterium compared with a non-modified strain when expression thereof is increased in the bacterium. Variants of the *ygaZH* genes and the *ychE* gene include homologues thereof. Homologues of the *ygaZH* genes and the *ychE* gene can be easily obtained from public databases by BLAST search or FASTA search using the aforementioned wild-type *ygaZH* genes and *ychE* gene of *Escherichia coli,* or wild-type *ychE* gene of *Pantoea ananatis* as a query sequence. Furthermore, homologues of the *ygaZH* genes and the *ychE* gene can also be obtained by PCR using the chromosome of an *Enterobacteriaceae* bacterium or a coryneform bacterium as a template and oligonucleotides prepared on the basis of these known gene sequences as primers.

The *ygaZH* genes and the *ychE* gene can each be a gene coding for a protein having the corresponding amino acid sequence mentioned above but including substitution, deletion, insertion, or addition of one or several amino acid residues at one or several positions, so long as they each codes for a protein having the activity of improving L-cysteine-producing ability of an *Enterobacteriaceae* bacterium compared with a non-modified strain when expression thereof is increased in the bacterium. In such a case, usually 70% or more, preferably 80% or more, more preferably 90% or more, of the activity of improving L-cysteine-producing ability of an *Enterobacteriaceae* bacterium compared with a non-modified strain when expression thereof is increased in the bacterium is maintained based on that of the protein not introduced with the substitution, deletion, insertion, or addition of one or several amino acid residues. Although the number meant by the term "one or several" can differ depending on the positions of amino acid residues in the three-dimensional structure of the protein or the types of amino acid residues, specifically, it is preferably 1 to 20, more preferably 1 to 10, still more preferably 1 to 5.

The above substitution, deletion, insertion, or addition of one or several amino acid residues is a conservative mutation that maintains normal function of the protein. The conservative mutation is typically a conservative substitution. The conservative substitution is a mutation wherein substitution takes place mutually among Phe, Trp and Tyr, if the substitution site is an aromatic amino acid; among Leu, Ile and Val, if it is a hydrophobic amino acid; between Gln and Asn, if it is a polar amino acid; among Lys, Arg and His, if it is a basic amino acid; between Asp and Glu, if it is an acidic amino acid; and between Ser and Thr, if it is an amino acid having hydroxyl group. Specific examples of conservative substitutions include: substitution of Ser or Thr for Ala; substitution of Gln, His or Lys for Arg; substitution of Glu, Gln, Lys, His or Asp for Asn; substitution of Asn, Glu or Gln for Asp; substitution of Ser or Ala for Cys; substitution of Asn, Glu, Lys, His, Asp or Arg for Gln; substitution of Gly, Asn, Gln, Lys or Asp for Glu; substitution of Pro for Gly; substitution of Asn, Lys, Gln, Arg or Tyr for His; substitution of Leu, Met, Val or Phe for Ile; substitution of Ile, Met, Val or Phe for Leu; substitution of Asn, Glu, Gln, His or Arg for Lys; substitution of Ile, Leu, Val or Phe for Met; substitution of Trp, Tyr, Met, Ile or Leu for Phe; substitution of Thr or Ala for Ser; substitution of Ser or Ala for Thr; substitution of Phe or Tyr for Trp; substitution of His, Phe or Trp for Tyr; and substitution of Met, Ile or Leu for Val. The above-mentioned amino acid substitution, deletion, insertion, addition, inversion etc. can be a result of a naturally-occurring mutation (mutant or variant) due to an individual difference, a difference of species, or the like of a bacterium from which the gene is derived.

Furthermore, the gene having such a conservative mutation as mentioned above can be a gene coding for a protein showing a homology of 80% or more, preferably 90% or more, more preferably 95% or more, still more preferably 97% or more, particularly preferably 99% or more, to the whole of the encoded amino acid sequence, and having the activity of improving L-cysteine-producing ability of an *Enterobacteriaceae* bacterium compared with a non-modified strain when expression thereof is increased in the bacterium. In this specification, "homology" can mean "identity".

Moreover, the *ygaZH* genes and the *ychE* gene can each be a DNA that is able to hybridize with a probe that can be prepared from a known gene sequence, for example, a sequence complementary to a part or the whole of the corresponding nucleotide sequence mentioned above, under stringent conditions, and coding for a protein having the activity of improving L-cysteine-producing ability of an *Enterobacteriaceae* bacterium compared with a non-modified strain when expression thereof is increased in the bacterium. The "stringent conditions" refer to conditions under which a so-called specific hybrid is formed, and a non-specific hybrid is not formed. Examples of the stringent conditions include those under which highly homologous DNAs hybridize to each other, for example, DNAs not less than 80% homologous, preferably not less than 90% homologous, more preferably not less than 95% homologous, still more preferably not less than 97% homologous, particularly preferably not less than 99% homologous, hybridize to each other, and DNAs less homologous than the above do not hybridize to each other, or conditions of washing of typical Southern hybridization, i.e., conditions of washing once, preferably 2 or 3 times, at a salt concentration corresponding to 1 x SSC, 0.1% SDS at 60°C, preferably 0.1 x SSC, 0.1% SDS at 60°C, more preferably 0.1 x SSC, 0.1% SDS at 68°C.

The probe used for the aforementioned hybridization can be a part of a sequence complementary to the gene as described above. Such a probe can be prepared by PCR using oligonucleotides prepared on the basis of a known gene sequence as primers and a DNA fragment containing the nucleotide sequence as a template. For example, when a DNA fragment having a length of about 300 bp is used as the probe, the washing conditions of the hybridization can be, for example, 50°C, 2 x SSC and 0.1% SDS.

The aforementioned explanations concerning variants of genes and proteins are also applied *mutatis mutandis* to arbitrary proteins including enzymes such as serine acetyltransferase and 3-phosphoglycerate dehydrogenase, and transporters such as the YdeD protein, and to the genes coding for them.

In the present invention, the phrase "expression of a gene is increased" means that the expression amount of an objective gene is increased compared with that observed in a non-modified strain such as a wild-type strain or parent strain. Although the degree of the increase of the expression amount of the gene is not particularly limited so long as the expression amount is increased compared with that observed in a non-modified strain, it is desirably increased 1.5 times or more, more preferably 2 times or more, still more preferably 3 times or more, compared with that observed in a non-modified strain. Furthermore, the case where "expression of a gene is increased" includes not only a case where the expression amount of an objective gene is increased in a strain in which the objective gene is natively expressed, but also a case where an objective gene is expressed in a strain in which the objective gene is not natively expressed. That is, for example, the case where "expression of a gene is increased" includes a case where an objective gene is introduced into a strain that does not have the objective gene, and expressed in the strain.

A modification for increasing expression of a gene can be attained by the methods for increasing expression of a gene exemplified in the section of <Method for increasing activity of protein> mentioned above.

Increase of the expression of a gene can be confirmed by, for example, confirming increase of the transcription amount of the gene by Northern hybridization or RT-PCR, or confirming increase of the amount of the protein by Western blotting, as described above. Increase of expression of the gene can also be confirmed by confirming increase of L-cysteine-producing ability, O-acetylserine resistance, or L-cysteine resistance.

### <2> Method for producing L-cysteine, related substance thereof, or mixture thereof of the present invention

When the bacterium of the present invention obtained as described above is cultured in a medium, L-cysteine, a related substance thereof, or a mixture thereof is accumulated in the medium. Therefore, by collecting the accumulated L-cysteine, the related substance thereof, or the mixture thereof from the medium, L-cysteine, the related substance thereof, or the mixture thereof can be produced.

Examples of the related substance of L-cysteine include L-cystine, derivatives of L-cysteine, and precursors of L-cysteine as described above. For example, as described later, when the bacterium of the present invention obtained as described above is cultured in a medium, O-acetylserine can be accumulated in the medium. In such a case, O-acetylserine can be produced by collecting O-acetylserine from the medium. O-acetylserine is one of amino acids, and is a precursor of L-cysteine biosynthesis. O-acetylserine has a chiral center, and therefore it can be used especially as a building block for synthesis of useful substances, and thus is useful. O-acetylserine can be produced alone, or can be produced in an arbitrary combination with L-cysteine, a related substance thereof, or a mixture thereof.

O-acetylserine is easily converted into N-acetylserine by a spontaneous reaction in a neutral to alkaline pH region. Therefore, when O-acetylserine is produced, it is preferable to prevent such conversion into N-acetylserine. Such conversion into N-acetylserine can be prevented by, for example, maintaining pH of culture medium to be in the acidic range (U.S. Patent Published Application No. 20020146783(A1)).

Examples of the medium to be used include ordinary media containing a carbon source, nitrogen source, sulfur source, inorganic ions, and other organic components as required.

As the carbon source, saccharides such as glucose, fructose, sucrose, glycerol, molasses, and starch hydrolysate, and organic acids such as fumaric acid, citric acid, and succinic acid can be used.

As the nitrogen source, inorganic ammonium salts such as ammonium sulfate, ammonium chloride, and ammonium phosphate, organic nitrogen such as soybean hydrolysate, ammonia gas, aqueous ammonia, and so forth can be used.

Examples of the sulfur source include inorganic sulfur compounds such as sulfates, sulfites, sulfides, hyposulfites, and thiosulfates.

Examples of the other organic components include organic trace amount nutrients. As organic trace amount nutrients, it is desirable to add required substances such as vitamin B₁, yeast extract, and so forth in appropriate amounts. Other than these, potassium phosphate, magnesium sulfate, iron ions, manganese ions, and so forth are added in small amounts, as required.

The culture is preferably performed under aerobic conditions for 30 to 90 hours. The culture temperature is preferably controlled to be at 25°C to 37°C, and pH is preferably controlled to be 5 to 8 during the culture. For pH adjustment, inorganic or organic acidic or alkaline substances, ammonia gas, and so forth can be used.

The product, i.e., L-cysteine, a related substance thereof, or a mixture thereof, can be collected from the medium after completion of the culture by a combination of ion-exchange resin method (Nagai, H. et al., Separation Science and Technology, 39(16), 3691-3710), membrane separation method (Japanese Patent Laid-open (Kokai) Nos. 9-164323 and 9-173792), crystallization method (WO2008/078448, W02008/078646), and other conventionally known methods.

The product collected in the present invention can contain cells of microorganism, medium components, moisture, and metabolic by-products of microorganism in addition to the objective substance. Purity of the product collected is usually 50% or higher, preferably 85% or higher, particularly preferably 95% or higher.

L-cysteine obtained as described above can be used for production of L-cysteine derivatives. The L-cysteine derivatives include methylcysteine, ethylcysteine, carbocisteine, sulfocysteine, acetylcysteine, and so forth.

Furthermore, when a thiazolidine derivative of L-cysteine is accumulated in the medium, L-cysteine can be produced by collecting the thiazolidine derivative from the medium and breaking the reaction equilibrium between the thiazolidine derivative and L-cysteine so that L-cysteine is produced in a larger amount. Furthermore, when S-sulfocysteine is accumulated in the medium, it can be converted into L-cysteine by reduction using a reducing agent such as dithiothreitol.

### Examples

Hereafter, the present invention will be explained more specifically with reference to an example.

### Example: L-cysteine production-increasing effect by enhancement of ygaZH genes or ychE gene expression

In order to investigate the effect of enhancement of expression of the *ygaZH* genes or the *ychE* gene on L-cysteine production in *Escherichia coli* and *Pantoea ananatis,* strains to which the *cysE5* gene coding for a mutant SAT desensitized to feedback inhibition by L-cysteine (U.S. Patent Published Application No. 20050112731) was introduced so that expression of the *ygaZH* genes or *ychE* gene was enhanced were constructed, and L-cysteine production culture was performed with them. The procedures are shown below,

### (1) Construction of expression plasmids

As expression vectors for enhancing expression of the *ygaZH* genes or the *ychE* gene, there were used pMIV-Pnlp23-ter (henceforth also referred to as pMIV-Pnlp23) and pMIV-Pnlp8-YeaS7 (henceforth also referred to as pMIV-Pnlp8), which were constructed from pMIV-5JS (Japanese Patent Laid-open (Kokai) No. 2008-99668). Construction methods of pMIV-Pnlp8 and pMIV-Pnlp23 are disclosed in Japanese Patent Laid-open (Kokai) No. 2010-187552. As a control, the empty vector pMIV-5JS (Japanese Patent Laid-open (Kokai) No. 2008-99668) was used.

pMIV-Pnlp23 contains a potent promoter Pnlp23 and the rrnB terminator, and contains *SalI* and *XbaI* sites between Pnlp23 and the rrnB terminator. Therefore, by using an objective gene designed to contain sites for the restriction enzymes *Sal*I and *Xba*I on the 5' and 3' end sides thereof, respectively, the objective gene can be cloned between the promoter and the terminator to construct an expression plasmid for the objective gene.

pMIV-Pnlp8 contains a potent promoter Pnlp8 and the rrnB terminator, and in this plasmid, the *yeaS* gene derived from the *E. coli* K12 strain is cloned between Pnlp8 and the rrnB terminator via *SalI* and *XbaI* sites. Therefore, by using an objective gene designed to contain sites for the restriction enzymes *SalI* and *XbaI* on the 5' and 3' end sides thereof, respectively, the *yeaS* gene fragment on the vector can be replaced with the objective gene fragment to construct an expression plasmid for the objective gene.

"Pnlp23" and "Pnlp8" are mutant type promoters for controlling expression intensity constructed from "Pnlp0", which is the promoter of the wild-type *nlpD* gene of the *E*. *coli* K12 strain. Pnlp8 and Pnlp23 provide different expression intensities (Pnlp8 > Pnlp23), and a promoter providing appropriate intensity can be chosen from them depending on a gene to be expressed.

Each of *ygaZH* (derived from *E. coli), ychE* (derived from *E. coli),* and *ychE* (derived from *P. ananatis)* was cloned into the aforementioned expression vector(s). As for *ygaZH,* since two genes *ygaZ* and *ygaH* forms an operon, the operon containing both genes was cloned into the expression vector. Cloning of each gene was performed by PCR using the genomic DNA of the *E. coli* MG1655 strain (ATCC 47076) or the *P. ananatis* SC17 strain (FERM BP-11091) as the template. The primers used are shown below: for *ygaZH* (derived from *E. coli),* ygaZH(Ec)SalI-F/ygaZH(Ec)XbaI-R (SEQ ID NOS: 1 and 2); for *ychE* (derived from *E. coli),* ychE(Ec)SalI-F/ychE(Ec)XbaI-R (SEQ ID NOS: 3 and 4); and for *ychE* (derived from *P. ananatis),* ychE(Pa)SalI-F/ychE(Pa)XbaI-R (SEQ ID NOS: 5 and 6). A site for the restriction enzyme *SalI* was designed in each of the upstream primers, and a site for the restriction enzyme *XbaI* was designed in each of the downstream primers. Therefore, the PCR product could be cloned into the expression plasmids pMIV-Pnlp8 and pMIV-Pnlp23 by using *SalI* and *Xba*I*.* In addition, the primers were designed so that the start codon GTG of *ychE* (derived from *E. coli)* and *ychE* (derived from *P. ananatis)* was replaced with ATG. PCR was performed by using PrimeSTAR HS DNA Polymerase (Takara) with a PCR cycle consisting of 94°C for 5 minutes, following 30 cycles of 98°C for 5 seconds, 55°C for 5 seconds, and 72°C for 90 seconds, and final incubation at 4°C. The PCR product was treated with *SalI* and *XbaI*, and ligated to pMIV-Pnlp8 or pMIV-Pnlp23 similarly treated with *Sal*I and *Xba*I to prepare an expression plasmid for each objective gene.

### (2) Construction of L-cysteine-producing bacterium

By introducing a mutant *cysE* coding for a mutant serine acetyltransferase of which feedback inhibition by L-cysteine is attenuated (*cysE5*, U.S. Patent Published Application No. 20050112731(A1)), an L-cysteine-producing bacterium that produces marked amounts of L-cysteine and O-acetylserine (OAS) can be constructed. Therefore, the plasmid pACYC-E1 carrying *cysE5* (Japanese Patent Laid-open (Kokai) No. 2010-207194) was introduced into the *Escherichia coli* MG1655 strain (ATCC 47076) and the *Pantoea ananatis* SC17 strain (FERM BP-11091) to construct an *E*. *coli* cysteine-producing bacterium and a *P. ananatis* cysteine-producing bacterium, respectively.

### (3) L-cysteine production culture

The expression plasmids for the objective genes were each introduced into the *E*. *coli* cysteine-producing bacterium and *P. ananatis* cysteine-producing bacterium constructed above. By using the obtained strains, culture for fermentative production of L-cysteine was performed, and the production amounts of L-cysteine were compared. For the culture, a production medium having the following composition was used.

### [L-cysteine production medium] (concentrations of the components are final concentrations)

| Components 1: | | |
|---|---|---|
| | (NH₄)₂SO₄ | 15 g/L |
| | KH₂PO₄ | 1.5 g/L |
| | MgSO₄ · 7H₂O | 1 g/L |
| | Thiamine hydrochloride | 0.1 mg/L |

| Components 2: | | |
|---|---|---|
| | FeSO₄ · 7H₂O | 1.7 mg/L |
| | Na₂MoO₄ · 2H₂O | 0.15 mg/L |
| | CoCl₂ · 6H₂O | 0.7 mg/L |
| | MnCl · 4H₂O | 1.6 mg/L |
| | ZnSO₄ · 7H₂O | 0.3 mg/L |
| | CuSO₄ · 5H₂O | 0.25 mg/L |

| Components 3: | | |
|---|---|---|
| | Tryptone | 0.6 g/L |
| | Yeast extract | 0.3 g/L |
| | NaCl | 0.6 g/L |

| Component 4: | | |
|---|---|---|
| | Calcium carbonate | 20 g/L |

| Component 5: | | |
|---|---|---|
| | L-Histidine hydrochloride monohydrate | 135 mg/L |

| Component 6: | | |
|---|---|---|
| | Sodium thiosulfate | 6 g/L |

| Component 7: | | |
|---|---|---|
| | Pyridoxine hydrochloride | 2 mg/L |

| Component 8: | | |
|---|---|---|
| | Glucose | 40 g/L |

For these components, there were prepared stock solutions of 10-fold concentration (Components 1), 1000-fold concentration (Components 2), 100/6-fold concentration (Components 3), 100-fold concentration (Component 5), 350/6-fold concentration (Component 6), 1000-fold concentration (Component 7), and 10-fold concentration (Component 8), they were mixed at the time of use, and the defined volume was obtained with sterilized water to obtain the final concentrations. Sterilization was performed by autoclaving at 110°C for 30 minutes (Components 1, 2, 3, 5, and 8), dry heat sterilization at 180°C for 5 hours or longer (Component 4), or filter sterilization (Components 6 and 7). In order to make the strains harbor the plasmids, 12.5 mg/L of tetracycline and 25 mg/L of chloramphenicol were added. For the culture of *E. coli,* the aforementioned medium was used. For the culture of *P. ananatis,* the aforementioned medium was used with the following modifications. That is, for the culture of *P. ananatis,* NH₄Cl was used instead of (NH₄)₂SO₄ of Component 1 as a nitrogen source at the same molar concentration in terms of ammonium ions, and the amount of sodium thiosulfate of Component 6 as a sulfur source was changed to 8 g/L.

The L-cysteine production culture was performed as follows. Each L-cysteine-producing strain was spread on the LB agar medium to perform pre-culture overnight at 34°C. Then the cells corresponding to about 7 cm on the plate were scraped once with an inoculation loop of 10 µl size (Blue Loop, NUNC), and inoculated into 2 ml of the production medium contained in a large test tube (internal diameter: 23 mm, length: 20 cm), so that the amounts of cells at the time of the start of the culture were adjusted to be substantially the same. Culture was performed at 32°C with shaking, and terminated after 23 hours (in the case of *E. coli)* or 30 hours (in the case of *P. ananatis*). L-cysteine (including L-cystine and derivatives of L-cysteine) produced in the medium was quantified by the method described by Gaitonde, M.K. (Biochem. J., 1967 Aug., 104(2):627-33).

Furthermore, since it is known that the L-cysteine secretion factor can also secrete O-acetylserine, the amount of O-acetylserine produced in the medium was also quantified. O-acetylserine was quantified by the following method. Since O-acetylserine is converted into N-acetylserine (NAS) under alkaline conditions, at first, the culture medium after completion of the culture was diluted 20 times with 200 mM Tris/HCl (pH 9.0) and left at room temperature, so that the whole produced O-acetylserine was converted into N-acetylserine (NAS). A sample prepared as described above was analyzed by HPLC (column, Inertsil ODS-3 (GL Science); flow rate, 1.0 ml/minute; column temperature, 40°C; detector, UV 210 mm; sample volume, 10 µl; buffer, 0.1 M K₂PO₄-H₃PO₄ (pH 2.2) and 5 mM sodium 1-octanesulfonate), and the concentration of O-acetylserine (including N-acetylserine) was calculated by comparison with the standard compound.

The experiment was performed in quadruplicate for each strain. Averages and standard deviations of the concentrations of L-cysteine and O-acetylserine produced in the medium are shown in Tables 1 to 4. In the tables, the concentrations of L-cysteine (including L-cystine and derivatives of L-cysteine) are shown in the column of "L-cysteine", and the concentrations of O-acetylserine (including N-acetylserine) are shown in the column of "OAS (NAS)". Furthermore, in the tables, the genes of which expression was enhanced are shown in the column of "Gene (organism as origin)", and "Vector" means the empty vector pMIV-5JS used as the control. Furthermore, combinations of the genes of which expression was enhanced and the promoters used are shown in Table 5 for the *E. coli* cysteine-producing bacterium and the *P. ananatis* cysteine-producing bacterium.

It was revealed that, as for the *E. coli* production bacterium, the amounts of accumulated L-cysteine and O-acetylserine were increased by enhancement of expression of *ygaZH* (derived from *E. coli*), *ychE* (derived from *E. coli*), or *ychE* (derived from *P. ananatis).* As for the *P. ananatis* production bacterium, it was also revealed that the amounts of accumulated L-cysteine and O-acetylserine were similarly increased by enhancement of expression of *ygaZH* (derived from *E. coli*), *ychE* (derived from *E. coli*), or *ychE* (derived from *P. ananatis*). From the results mentioned above, it was revealed that enhancement of expression of *ygaZH* or *ychE* was effective for production of L-cysteine (including L-cystine and derivatives of L-cysteine) and O-acetylserine (including N-acetylserine).

[Table 1]

**Table 1: L-cysteine production by E. coli production bacterium**

| Gene (organism as origin) | L-cysteine (g/L) |
|---|---|
| Vector | 0.29 ± 0.05 |
| *ygaZH* (*E. coli*) | 0.37 ± 0.10 |
| *ychE* (*E. coli*) | 0.91 ± 0.04 |
| *ychE* (*P. ananatis*) | 0.94 ± 0.08 |

[Table 2]

**Table 2: O-acetylserine production by E. coli production bacterium**

| Gene (organism as origin) | OAS (NAS) (g/L) |
|---|---|
| Vector | 0.61 ± 0.15 |
| *ygaZH* (*E. coli*) | 0.87 ± 0.23 |
| *ychE* (*E. coli*) | 3.95 ± 2.37 |
| *ychE* (*P. ananatis*) | 6.97 ± 1.05 |

[Table 3]

**Table 3: L-cysteine production by P. ananatis production bacterium**

| Gene (organism as origin) | L-cysteine (g/L) |
|---|---|
| Vector | 0.26 ± 0.04 |
| *ygaZH* (*E. coli*) | 0.75 ± 0.02 |
| *ychE* (*E. coli*) | 0.46 ± 0.02 |
| *ychE* (*P. ananatis*) | 0.49 ± 0.10 |

[Table 4]

**Table 4: O-acetylserine production by P. ananatis production bacterium**

| Gene (organism as origin) | OAS (NAS) (g/L) |
|---|---|
| Vector | 0.62 ± 0.05 |
| *ygaZH* (*E. coli*) | 1.45 ± 0.07 |
| *ychE* (*E. coli*) | 1.37 ± 0.09 |
| *ychE* (*P. ananatis*) | 1.33 ± 0.11 |

[Table 5]

**Table 5: Combinations of expression-enhanced gene and promoter**

| Gene (organism as origin) | *E. coli* production bacterium | *P. ananatis* production bacterium |
|---|---|---|
| *ygaZH* (*E. coli*) | Pnlp8 | Pnlp8 |
| *ychE* (*E. coli*) | Pnlp8 | Pnlp23 |
| *ychE* (*P. ananatis*) | Pnlp23 | Pnlp23 |

### Industrial Applicability

According to the present invention, L-cysteine-producing ability of a bacterium belonging to the family *Enterobacteriaceae* can be improved. Furthermore, by using the bacterium of the present invention, L-cysteine, a related substance thereof, or a mixture thereof can be efficiently produced by fermentation.

### Explanation of Sequence Listing

SEQ ID NOS: 1 to 6, Primers
SEQ ID NO: 7, Nucleotide sequence of *E. coli ygaZ* gene
SEQ ID NO: 8, Amino acid sequence of *E. coli* YgaZ
SEQ ID NO: 9, Nucleotide sequence of *E. coli ygaH* gene
SEQ ID NO: 10, Amino acid sequence of *E. coli* YgaH
SEQ ID NO: 11, Nucleotide sequence of *E. coli ygaZH* operon
SEQ ID NO: 12, Nucleotide sequence of *E. coli ychE* gene
SEQ ID NO: 13, Amino acid sequence of *E.* coli YchE
SEQ ID NO: 14, Nucleotide sequence of *P. ananatis ychE* gene
SEQ ID NO: 15, Amino acid sequence of *P. ananatis* YchE
SEQ ID NO: 16, Nucleotide sequence of *E. coli* wild-type *cysE*
SEQ ID NO: 17, Amino acid sequence of *E. coli* wild-type CysE

## Claims

1. A method for producing L-cysteine, a related substance thereof, or a mixture thereof, comprising:
culturing a bacterium belonging to the family *Enterobacteriaceae,* which has L-cysteine-producing ability and is modified so that expression of gene(s) selected from the group consisting of *ygaZH* genes and *ychE* gene is increased compared with a non-modified strain, in a medium, and
collecting L-cysteine, a related substance thereof, or a mixture thereof from the medium.

2. The method according to claim 1, wherein the bacterium is modified so that the L-cysteine biosynthesis system is enhanced.

3. The method according to claim 1 or 2, wherein the bacterium has a mutant serine acetyltransferase of which feedback inhibition by L-cysteine is attenuated or eliminated.

4. The method according to any one of claims 1 to 3, wherein the *ygaZH* genes code for a protein selected from the group consisting of (A) and (B) mentioned below, and a protein selected from the group consisting of (C) and (D) mentioned below, respectively:
(A) a protein having the amino acid sequence of SEQ ID NO: 8;
(B) a protein having the amino acid sequence of SEQ ID NO: 8 but which includes 1 to 10 amino acid substitutions, deletions, insertions, or additions, and having an activity of improving L-cysteine-producing ability of the Enterobacteriaceae bacterium compared with a non-modified strain when expression thereof is increased in the bacterium;
(C) a protein having the amino acid sequence of SEQ ID NO: 10;
(D) a protein having the amino acid sequence of SEQ ID NO: 10 but which includes 1 to 10 amino acid substitutions, deletions, insertions, or additions, and having an activity of improving L-cysteine-producing ability of the Enterobacteriaceae bacterium compared with a non-modified strain when expression thereof is increased in the bacterium.

5. The method according to any one of claims 1 to 4, wherein the *ychE* gene codes for a protein selected from the group consisting of (E) and (F) mentioned below: (E) a protein having the amino acid sequence of SEQ ID NO: 13 or 15;
(E) a protein having the amino acid sequence of SEQ ID NO: 13 or 15 but which includes 1 to 10 amino acid substitutions, deletions, insertions, or additions, and having an activity of improving L-cysteine-producing ability of the Enterobacteriaceae bacterium compared with a non-modified strain when expression thereof is increased in the bacterium.

6. The method according to any one of claims 1 to 5, wherein the *ygaZH* genes are a DNA selected from the group consisting of (a) and (b) mentioned below, and a DNA selected from the group consisting of (c) and (d) mentioned below, respectively:
(a) a gene having the nucleotide sequence of SEQ ID NO: 7;
(b) a DNA that hybridizes with a sequence complementary to the nucleotide sequence of SEQ ID NO: 7 or a probe that can be prepared from the nucleotide sequence under stringent conditions, and codes for a protein having an activity of improving L-cysteine-producing ability of the Enterobacteriaceae bacterium compared with a non-modified strain when expression thereof is increased in the bacterium;
(c) a gene having the nucleotide sequence of SEQ ID NO: 9;
(d) a DNA that hybridizes with a sequence complementary to the nucleotide sequence of SEQ ID NO: 9 or a probe that can be prepared from the nucleotide sequence under stringent conditions, and codes for a protein having an activity of improving L-cysteine-producing ability of the Enterobacteriaceae bacterium compared with a non-modified strain when expression thereof is increased in the bacterium.

7. The method according to any one of claims 1 to 6, wherein the *ychE* gene is a DNA selected from the group consisting of (a) and (b) mentioned below:
(a) a gene having the nucleotide sequence of SEQ ID NO: 12 or 14;
(b) a DNA that hybridizes with a sequence complementary to the nucleotide sequence of SEQ ID NO: 12 or 14 or a probe that can be prepared from the nucleotide sequence under stringent conditions, and codes for a protein having an activity of improving L-cysteine-producing ability of the *Enterobacteriaceae* bacterium compared with a non-modified strain when expression thereof is increased in the bacterium.

8. The method according to any one of claims 1 to 7, wherein expression of the gene(s) is increased by increasing the copy number of the gene(s), or by modifying an expression control sequence of the gene(s).

9. The method according to any one of claims 1 to 8, wherein the bacterium is a bacterium belonging to the genus *Pantoea.*

10. The method according to claim 9, wherein the bacterium is *Pantoea ananatis.*

11. The method according to any one of claims 1 to 8, wherein the bacterium is a bacterium belonging to the genus *Escherichia.*

12. The method according to claim 11, wherein the bacterium is *Escherichia coli.*

13. The method according to any one of claims 1 to 12, wherein the related substance is L-cystine or a thiazolidine derivative.

14. The method according to any one of claims 1 to 12, wherein the related substance is O-acetylserine.
